Europäisches Patentamt

(19) European Patent Office

Office européen des brevets

(11) EP 0 892 068 A1

(12) EUROPEAN PATENT APPLICATION

(43) Date of publication:
20.01.1999 Bulletin 1999/03

(51) Int. Cl.$^6$: C12Q 1/68

(21) Application number: 97401740.2

(22) Date of filing: 18.07.1997

(84) Designated Contracting States:
AT BE CH DE DK ES FI FR GB GR IE IT LI LU MC NL PT SE

(71) Applicant: Genset
75008 Paris (FR)

(72) Inventors:
• Cohen, Daniel
94120 Fontenay-Sous-Bois (FR)

• Blumenfeld, Marta
75013 Paris (FR)

(74) Representative: Warcoin, Jacques
Cabinet Régimbeau,
26, avenue Kléber
75116 Paris (FR)

(54) **Method for generating a high density linkage disequilibrium-based map of the human genome**

(57) Methods for generating a high density linkage disequilibrium map of the human genome, markers obtained by the said methods, probes capable of hybridising with the said markers, and primers capable of detecting the said markers, oligonucleotide arrays comprising sets of the said probes or primers, diagnostic assay using the said probes and genes identified by the said methods.

EP 0 892 068 A1

**Description**

This invention relates to methods for generating a high density linkage disequilibrium map of the human genome, markers obtained by the said methods, probes capable of hybridising with the said markers, diagnostic assay using the said probes and genes identified by the said methods.

Background of the invention

Analysing the human genome

The first step of the international cooperative venture to analyse the human genome has been the construction of genetic and physical maps. Genetic maps represent the position of polymorphic loci along the chromosomes whereas physical maps are collections of ordered overlapping cloned fragments of genomic DNA, together with a specification of their arrangement along the chromosomes. Genetic and physical maps have proved essential to identify genes which are involved in diseases, or in other important traits.

The human haploid genome contains an estimated 80,000 to 100,000 genes scattered on a $3 \times 10^9$ base-long double stranded DNA. Each human being is diploid, *i.e.* possesses two haploid genomes, one from paternal origin, the other from maternal origin. The sequence of the human genome varies among individuals in a population. About $10^7$ sites scattered along the $3 \times 10^9$ base pairs of DNA are polymorphic, existing in at least two variant forms called alleles. Most of these polymorphic sites are generated by single base substitution mutations and are bi-allelic. Less than $10^5$ polymorphic sites are due to more complex changes and are very often multiallelic, i.e. exist in more than two allelic forms. At a given polymorphic site, any individual (diploid), can be either homozygous (twice the same allele) or heterozygous (two different alleles). A given polymorphism or rare mutation can be either neutral (no effect on phenotype), or functional, *i.e.* responsible for a particular genetic trait.

It is worth noting that traits can either be "binary", e.g. diabetic vs. non diabetic, or "quantitative", e.g. elevated blood pressure. Individuals affected by a quantitative trait can be classified according to an appropriate scale of trait values, e.g. blood pressure ranges. Each trait value range can then be analysed as a binary trait: patients showing trait value within one such range will be studied in comparison with patients showing trait value out of this range. In such a case, genetic analysis methods will be applied to subpopulations of individuals showing trait values within defined ranges.

The ultimate goals of the human genome project are :

- the comprehensive sequencing of the 3 billion base pairs of DNA which the human genome is made of,
- the identification of the estimated 80,000 to 100,000 genes spanned over the human genome,
- the understanding of the involvement of these genes, and their different alleles, in human diseases, as well as the characterisation of gene interactions therein, and
- the understanding of the involvement of these genes, and their different alleles, in other complex traits such as the response to drug treatment or to environmental factors.

Genetic maps

The first step towards the identification of genes involved in a particular genetic trait (a disease or any other important trait) consists in the localisation of genomic regions containing trait-causing genes, by means of genetic mapping methods. Genetic mapping involves the analysis of the segregation of polymorphic loci in trait positive and trait negative populations. Polymorphic loci constitute a small fraction of the human genome (less than 1%), compared to the vast majority of human genomic DNA which is identical in sequence among the chromosomes of different individuals. Among all existing human polymorphic loci, genetic markers can be defined as genome-derived polynucleotides which are sufficiently polymorphic to allow a reasonable probability that a randomly selected person will be heterozygous, and thus informative for genetic analysis by methods such as linkage analysis or association studies, which methods are described below.

A genetic map consists of an ordered collection of genetic markers. The optimal genetic map should present the following characteristics:

- the density of the genetic markers scattered along the genome should be sufficient to allow the identification and localisation of any trait-related polymorphism,

- each marker should have an adequate level of heterozygosity, so as to be informative in a large percentage of different meioses,
- all markers should be easily typed on a routine basis, at a reasonable expense, and in a reasonable amount of time,

- the entire set of markers per chromosome should be ordered in a highly reliable fashion.

The invention provides such a map based on a collection of bi-allelic markers of the human genome.

The analysis of DNA polymorphisms has relied on genetic markers which can be classified in the following three categories:

- RFLPs :Restriction Fragment Length Polymorphisms were the first generation genetic markers. They are single nucleotide polymorphisms which occur at restriction sites, therefore modifying the cleavage pattern of the corresponding restriction enzyme. Though the original methods used to type RFLPs were material-, effort- and time-consuming, today these markers can easily be typed by PCR-based technologies. Since they are bi-allelic markers (they present only two alleles, the restriction site being either present or absent), their maximum heterozygosity is 0.5. The potential number of RFLPs spanned along the entire genome is more than $10^5$, which leads to a theoretical average inter-marker distance of 30 kilobases. However, the number of evenly distributed RFLPs which would be sufficiently informative to allow the tracking of genetic polymorphisms turned out to be very limited.

- VNTRs : a second generation series of genetic markers is composed of the so-called DNA VNTRs, for Variable Number of Tandem Repeats. On the one hand, minisatellites form a collection of tandemly repeated DNA sequences which are dispersed along considerable portions of the human genome, ranging from 0.1 to 20 kilobases. Since they present many possible alleles, their polymorphic informative content is very high ; however, there are only $10^4$ potential VNTRs that can be typed by Southern blotting. On the other hand, microsatellites (also called simple tandem repeat polymorphisms, or simple sequence length polymorphisms) constitute the most developed category of genetic markers : they include small arrays of tandem repeats of simple sequences (di-tri-tetra- nucleotides repeats), which exhibit a high degree of length polymorphism, and thus a high level of informativeness. Only just more than 5,000 microsatellites (out of the $10^4$ VNTRs), easily typed by PCR-derived technologies, have been ordered along the human genome (Dib et al., 1996).

The former markers contributed to the establishment of the first (RFLPs) and second (microsatellites) generation genetic maps, which comprised from 400 to the currently used 5,000 markers. However, the limited number of publicly available informative markers that have revealed accessible and easily typed implied that the average distance between two such markers remained excessive to allow the successful accomplishment of the above listed challenges.

Single Nucleotide Bi-allelic Markers

Bi-allelic markers are genome-derived polynucleotides which exhibit bi-allelic polymorphism at one single base position. By definition, the lowest allele frequency of a bi-allelic polymorphism is 1%; sequence variants which show allele frequencies below 1% are called rare mutations. There are potentially more than $10^7$ bi-allelic markers which can easily be typed by routine automated techniques, such as sequence- or hybridisation-based techniques. However, a bi-allelic marker will show a sufficient degree of informativeness for genetic mapping only provided the frequency of its less frequent allele is not less than about 0.3, i.e. its heterozygosity rate is higher than about 0.42 ( the heterozygosity rate for a bi-allelic marker is 2 $P_a$ (1-$P_a$) , where $P_a$ is the frequency of allele a).

Although these are the most abundant type of genetic markers present throughout the human genome, the generation of a genome-wide bi-allelic marker map requires an enormous effort: such markers have to be selected in sufficient numbers, each of them has to present a sufficient degree of informativeness, and the whole set has to be evenly distributed along the genome. Despite the recently reinforced interest in the Human Genome Project, such a task remains an unresolved challenge, and no adequate technological strategy has been proposed up to today.

Existing genome-wide maps

All existing genome-wide genetic maps have been built in two steps: first, the random generation and selection of polymorphic markers, and second, their ordering along the human genome.

In order to generate the markers, random genetic sites have been tested for polymorphism by analysing 5 to 10 individuals. Various methods have been used, such as amplicon restriction fragment length polymorphism (RFLP detection), amplicon length polymorphism (detection of microsatellites), amplicon conformation polymorphism, or amplicon sequencing (detection of bi-allelic markers other than RFLPs).

In order to sequentially order the obtained markers, genetic methods were used (linkage by genotyping the same set of reference families), as well as physical methods (radiation hybrids) (Benham et al., 1989; Cox et al., 1990).

Today's available maps of the human genome are based only on the microsatellite type of genetic markers:

- CEPH's YAC map contains 2601 polymorphic Sequences Tag Sites (STSs) (Chumakov et al., 1995), and is an integrated physical and genetic map which covers 75% of the genome;
- WhiteHead Institute and Généthon's map comprises 15,086 STSs (Hudson et al., 1995), and is also and integrated physical and genetic map, covering 95% of the genome;
- Généthon's map containing 5,264 genetic markers (Dib et al., 1996) is a genetic map;
- Généthon and Cambridge University's Radiation Hybrid map containing 850 Sequenced Tag Sites (STSs) (Gyapay et al., 1994) is a genetic map.

The methods used to generate these maps did not allow the resulting selection of markers to be evenly distributed along the genome. A characteristic of the invention is the generation of a set of informative, polymorphic markers evenly and densely distributed along the entire human genome.

## Genetic mapping methods: Linkage Analysis

First and second generation genetic maps were constructed in order to enable genetic linkage analysis: this has been the main statistical approach successfully used up to now to identify trait-related genes.

Linkage analysis aims at establishing a correlation between the transmission of genetic markers and that of a specific trait throughout generations within a family.

The procedure is the following . All members of a series of affected families are genotyped with a set of markers (a few hundred ; one every 10 Mb). By comparing genotypes in all members, one can attribute sets of alleles to parental haploid genomes (haplotyping or phase determination). The origin of recombined fragments is then determined in the offspring of all families. Those which co-segregate with the trait are tracked. Statistics are performed after pooling data from all families. As a result of the statistical linkage analysis, one or several regions are selected as candidate regions, based on their high probability (lod score) to carry a trait causing allele.

Using a second generation genetic map (comprising over 5,000 microsatellite markers), linkage analysis enables the localisation of disease genes within chromosomal regions of ca. 2 cM - 20 cM length. This approach has proved efficient for simple genetic traits with high penetrance trait causing alleles at a few loci. The penetrance of a trait causing allele $\underline{a}$ is defined as the ratio between the number of trait positive $\underline{a}$ carriers and the total number of $\underline{a}$ carriers within the population. About 100 pathological trait causing genes were discovered by linkage analysis over the last 10 years. In most of these cases, the majority of affected individuals had affected relatives and the pathological trait was rare in the population ( with a frequency lower than 0.1 %). In about 10 cases, the pathological trait was more common, but the discovered mutated gene was very rare in the affected population (Alzheimer's Disease, Breast cancer, Type II Diabetes): these genes revealed not to be responsible for the trait in sporadic cases.

The major drawbacks of the linkage analysis method include:

- its sensitive reliance on the choice of a genetic model suitable to each studied trait
- the limits on the ultimate resolution attainable, and the need to further implement complementary studies in order to refine the analysis of genomic regions often in the range of 2 to 20 Mb
- the effort and cost needed for the recruitment of suitable informative families, in adequate numbers for the study to be successfully conducted.

Finally, due to the complexity of most genetic traits, linkage analysis has serious limitations :

- It has limited power to detect low penetrance trait causing alleles involved in complex genetic traits, and too large an effort to collect affected families is required for applying linkage analysis to these situations (Risch and Merikangas, 1996). This is essentially on the one hand because more independent trait causing genes being involved in complex traits, more families are required to obtain a good probability of linkage, and on the other hand because low penetrance generates background noise in linkage studies since very often, a trait causing allele carrier is not affected.
- It cannot be applied to the study of traits for which no available large informative families are available; typically, this will be the case in any attempt to identify trait causing alleles involved in sporadic cases. An important example of such a sporadic trait is the response to a drug treatment.

## Genetic mapping methods: Association studies

The best alternative to map susceptibility genes for sporadic traits is to look for statistical associations between the trait and some marker genotype when comparing a case (trait [+] ) and a control (trait [-] ) population.

The rational of this approach is to select candidate genes potentially involved in the pathological pathway of interest, then to search for polymorphisms in those genes, and finally to detect if these polymorphisms (alleles) are more frequent in an unrelated trait [+] population than in an unrelated trait [-] or random population. This candidate gene approach, provided the samples are large enough and the genetic background of the tested population is well known, may be a valuable analysis tool (as shown in the cases of apolipoprotein (Apo) E e4 allele and late onset Alzheimer's Disease ; HLA DR3/DR4 alleles and Type I Diabetes ; HLA B27 allele and ankylosing spondylitis ; angiotensin-converting enzyme (ACE) D allele and coronary atherosclerosis/myocardial infarction ; angiotensinogen (AGT) M235T allele and essential hypertension) (Lathrop M., 1993). However, in order to validate the results provided by a candidate gene approach, its interpretation must take into account the phenomenon of linkage disequilibrium (LD).

LD is defined as the trend for alleles at nearby loci on haploid genomes to correlate in the population. For example, $\underline{a}$ and $\underline{b}$, alleles at close loci A and B, are said to be in linkage disequilibrium if the $\underline{a}\,\underline{b}$ haplotype (a haplotype is defined as a set of alleles on the same chromosomal segment) has a frequency which is statistically higher than $P_a \times P_b$ (expected frequency if the alleles segregate independently, where $P_a$ is the frequency of allele $\underline{a}$, and $P_b$ that of allele $\underline{b}$).

Due to LD, assignment of a candidate allele as a trait causing allele based only on the analysis of its frequency without assessing the frequency of flanking polymorphisms could be misleading : the putative candidate allele may not be the trait-causing allele, but instead an allele being in LD with the actual trait causing allele. For this reason, in order to correctly exploit candidate gene association studies, for each candidate gene which is analysed for potential association with a trait, flanking polymorphisms must also be assessed to fully validate the results.

Even though genome-wide candidate gene association studies could potentially be more powerful than linkage analysis, this approach is not feasible at present, since all functional polymorphisms ($10^6$, approximately 10% of total biallelic polymorphisms) should be tested and only a few hundred are actually known.

It has recently been suggested (Risch and Merikangas, 1996) that taking advantage of linkage disequilibrium may allow to reduce the number of genetic markers and genotyping tests needed to implement genetic mapping through association studies. However having the technological capacity and tools to develop a third generation map comprising a large number of bi-allelic markers, and to achieve genome-wide association studies still remains an unresolved problem. A particular embodiment of this invention is a method to generate adequate high density genetic maps of the human genome, that would enable such studies to be run.

Suggested strategies for the generation of high density maps

The most recent approaches to develop third generation maps based on bi-allelic polymorphisms entail the identification of single nucleotide polymorphisms within arrays of STSs (Sequenced Tag Sites) selected among the available ca. 30,000 STSs (Hudson et al., 1995; Schuler et al., 1996).

Wang et al. (1997) recently announced the identification and mapping of 750 Single Nucleotide Polymorphisms issued from the sequencing of 12,000 STSs from the Whitehead/MIT map, in eight unrelated individuals. The work has been carried through a high throughput system based on the utilisation of the DNA chips technology from Affymetrix (Chee et al., 1996).

According to experimental data and statistical calculations, only less than one out of 10 from all STSs mapped today may contain an informative Single Nucleotide Polymorphism. This is mainly due to the short length of existing STSs (usually less than 250 bp) : if one assumes $10^6$ informative polymorphisms spread along the human genome, there would on average be one marker of interest every $3.10^9/10^6$, i.e. every 3,000 bp. The probability that one such marker is present on a 250 bp stretch is thus less than 1/10. While the above proposed approach may enable the generation of a high density map, this however would assume the prior sequencing and localisation of numerous additional STSs . Moreover, this approach, based on existing markers, does not as such consider putting any systematic effort into making sure that the markers obtained will be optimally distributed throughout the entire genome.

The even distribution of markers along the chromosomes is key to the future success of genetic analyses addressing the challenges described above, especially association studies on sporadic cases. Yet, to generate a high density map of bi-allelic markers evenly distributed along the genome, and to then perform genotyping studies based on the above mentioned attempts, will imply redhibitory efforts, in terms of technology, material, time and cost.

This invention presents a method to generate a high density linkage disequilibrium-based map of the human genome, which will allow the identification of markers and genes, particularly those involved in sporadic traits, and which uses the concepts of genome-wide association studies and linkage disequilibrium mapping.

The present invention relates to methods for generating a high density linkage disequilibrium map of the human genome, comprising the steps of:

a) ordering a set of 10,000 to 20,000 cloned genomic fragments along the human genome, with average size ranging from 100 kb to 300 kb;
b) generating several bi-allelic markers per fragment; and
c) selecting one to three bi-allelic marker per fragment, with heterozygosity rate higher than 40%.

The present invention also relates to methods for generating a high density linkage disequilibrium map of the human genome, comprising the steps of:

a) ordering a set of 15,000 to 20,000 BACs along the human genome, with average insert size ranging from 100 kb to 200 kb;
b) generating several bi-allelic markers per BAC; and
c) selecting one to three bi-allelic marker per BAC, with heterozygosity rate higher than 40%.

In a preferred embodiment, the invention is directed to methods according to the invention where bi-allelic markers are preferably generated in any region with no evidence of linkage disequilibrium.

In another preferred embodiment, the invention is also directed to methods according to the invention where bi-allelic markers are preferably generated in any region with evidence for a positive association with a genetic trait.

The invention also relates to a map of the human genome obtained by a method according to the invention.

The invention comprises a subset of markers derived from a map according to the invention.

The invention also comprises bi-allelic markers obtained by a method according to the invention.

It is another object of the present invention to provide methods of identifying one or several bi-allelic markers associated with a trait, comprising the steps of:

a) scanning groups of markers according to the invention in trait $^+$ and trait $^-$ individuals; and
b) establishing a statistically significant association between one allele of the marker(s) and the trait.

The invention also provides methods of identifying a gene associated with a trait, comprising the steps of:

a) identifying one or several marker(s) using a method according the invention; and
b) establishing a statistically significant association between one or several allele(s) of a gene in the vicinity of the identified marker(s) and the trait.

In a preferred embodiment, the invention relates to methods according to these above methods where said trait is a disease or a drug response.

The invention also relates to methods according to the invention where said drug response is efficacy, toxicity and/or tolerance.

The invention comprises markers obtained by a method according to the invention.

The invention further relates to oligonucleotide probes comprising a sequence capable of hybridising specifically with one allele of a marker according to the invention.

In a preferred embodiment, the invention is directed to oligonucleotide probes capable of hybridising specifically with the sequence of one marker's allele identified by a method according to the invention.

In another preferred embodiment, the invention is directed to oligonucleotide primers capable of specifically detecting the sequence of one marker's allele identified by a method according to the invention.

It is another object of the present invention to provide high density oligonucleotide arrays comprising a subset of marker probes or primers from a map according to the invention. Such arrays can be obtained by synthesis and/or immobilisation of said subset of marker probes or primers on any appropriate support. Immobilisation of large numbers of oligonucleotides on such supports as glass and silicium can be achieved by mechanical distribution or electric or magnetic addressing to specific locations on these supports. Alternatively, parallel synthesis of large numbers of markers can be achieved directly on the support by using appropriate techniques, such as photolithography.

It is another object of the present invention to provide diagnostic assays using an oligonucleotide probe according to the invention.

The oligonucleotide probes according to the invention can be preliminary labelled before use, for example radiolabelled, chemilumiscentlabelled, fluorescentlabelled or enzymlinked probes.

Preferably the oligonucleotide probes and primers according to the invention comprise at least 10 nucleotides. Among the shortest probes which contain about 10 to 20 nucleotides, the suitable conditions for hybridization correspond to stringence conditions which are normally used in standard methods, described for example in the experimental procedure.

In a preferred embodiment, the invention comprises diagnostic assays according to the invention, where said probe is immobilised on a solid support.

According to the invention, the probes can be fixed on solid support. Said solid supports, which are well known for screening using oligonucleotide probes in diagnosis or pharmaceutical discovery area, comprise for example, but are not limited to, polymeric support, such as polystyren, polyethylen, polypropylen, polyamides, cellulose, and their derived or silicium support or glass.

Furthermore, the present invention relates to genes associated with a trait which are identified by methods according to the invention. According to the invention, it is understood that *genes* will be isolated following standard laboratory protocols.

Finally, the invention relates to methods for sequencing nucleic acid of said genes according to the invention, comprising the step of using probe or primer according to the invention.

Legend of the figures

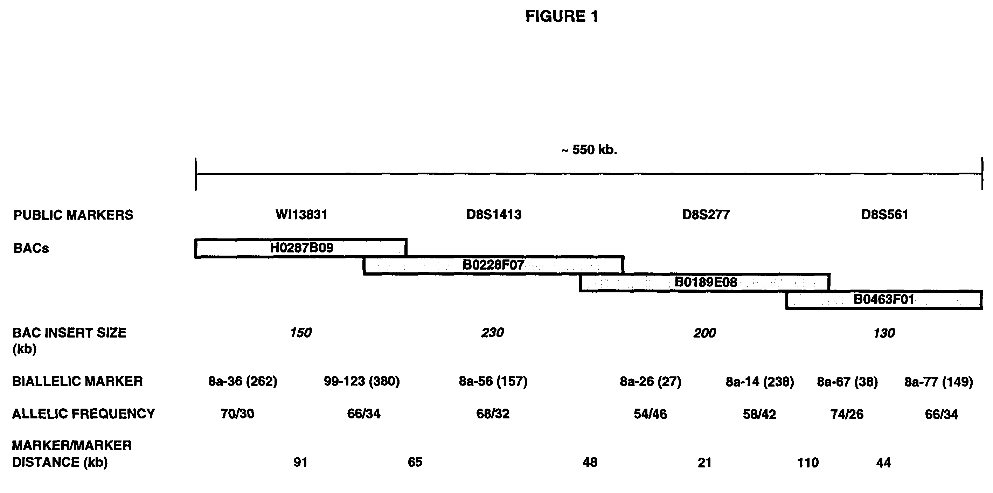

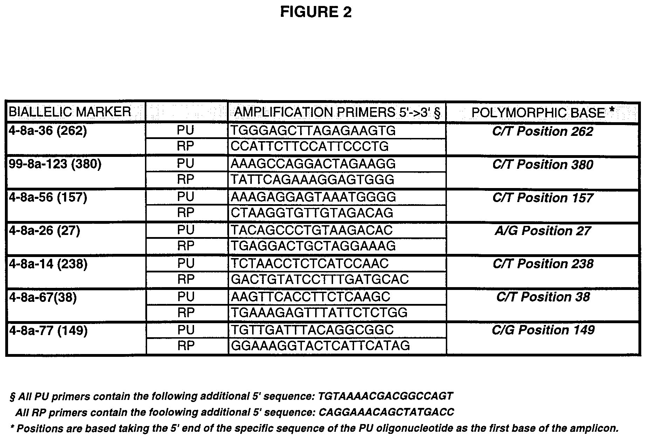

Figure 1 shows a bi-allelic marker map of a region spanning 500kb in chromosome 8p23. The seven bi-allelic markers were generated as described in Example 4. The particular STSs that were screened in order to isolate the BAC clones which were used to generate the bi-allelic markers are indicated as Public Markers. PCR primers used for the amplification of the bi-allelic markers are depicted in Figure 2. Bi-allelic markers were obtained by sequencing amplification products derived from a pool of 100 unrelated individuals corresponding to a French heterogeneous population. Allelic frequencies of the bi-allelic markers were determined by microsequencing the same 100 DNA samples mentioned above, as described in Example 5.

Figure 2 shows the sequence of the oligonucleotide primers which allow to amplify the bi-allelic markers described in Figure 1. The position of the polymorphic base in each bi-allelic marker is indicated by giving the position of the variable nucleotide in the corresponding amplicon, considering the 5' end of the specific sequence of the PU oligonucleotide - thus, not including the PU/RP sequencing tails - as the first base of the amplicon.

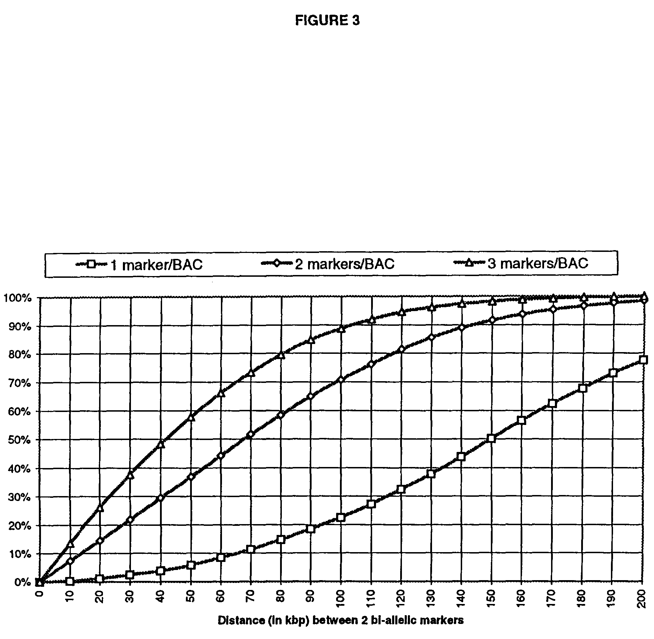

Figure 3 illustrates a computer simulation of the distribution of inter-marker spacing, on a randomly distributed bi-allelic marker set, depending on the total density of the generated genetic map. One hundred iterations were performed for each simulation (20,000 marker map, 40,000 marker map, 60,000 marker map).

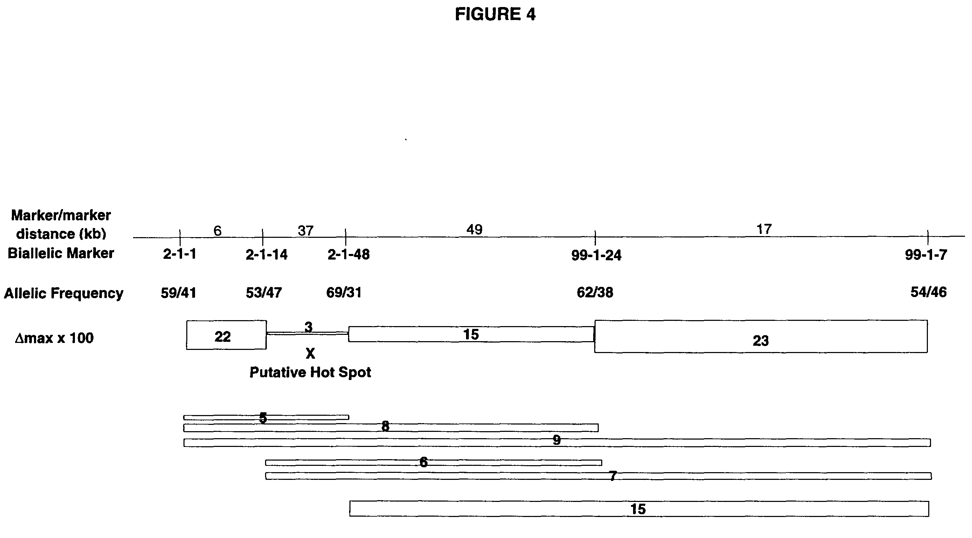

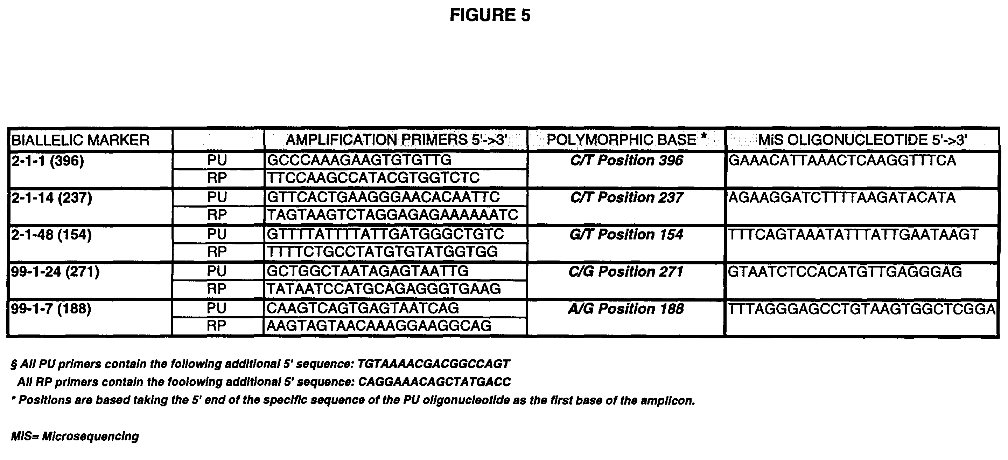

Figure 4 illustrates the identification of a putative recombinational hot spot in the 1q21 human genomic region. BAC 123H04M, harbouring this chromosomal region, was isolated by BAC screening procedures described in example 2, using STS D1S3423 (WI-10286). 5 bi-allelic markers were generated from BAC 123H04M and genotyped in the French population defined in Figure 1, using the oligonucleotides described in Figure 5. Linkage disequilibrium (Δ max) was measured using the Piazza formula (see example 6).

Figure 5 shows the sequence of the oligonucleotide primers which allow to amplify and genotype the bi-allelic markers described in Figure 4. Genotyping is performed by running microsequencing reactions on DNA samples from the French population defined in Figure 1.

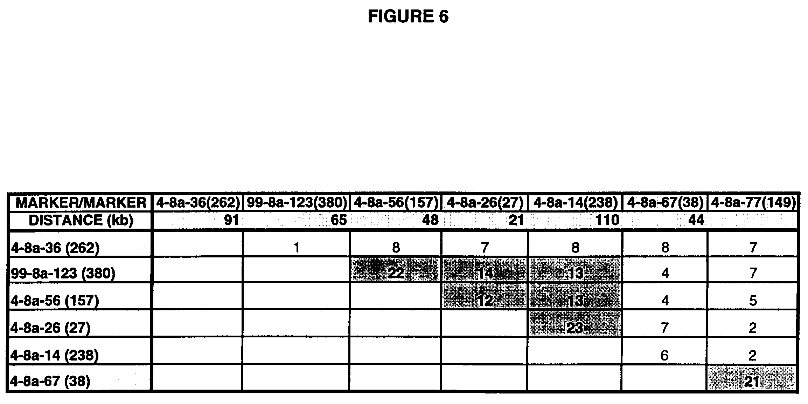

Figure 6 is a matrix representation of linkage disequilibrium analysis of the ca. 500 kb region of chromosome 8 described in Figure 1. Genotyping is performed by running microsequencing reactions on DNA samples from the French population defined in Figure 1. Disequilibrium values were calculated using a software implementing the Piazza formula approach. Values shown represent Δmax x 100.

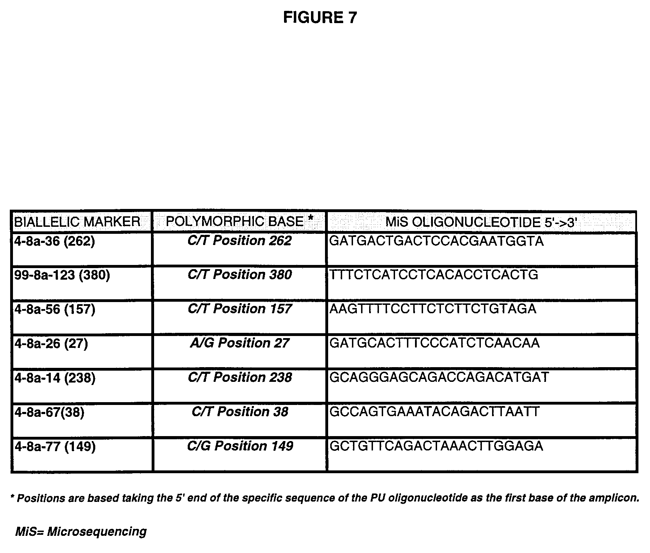

Figure 7 describes the oligonucleotides used to perform the genotyping of markers analysed in Figure 6.

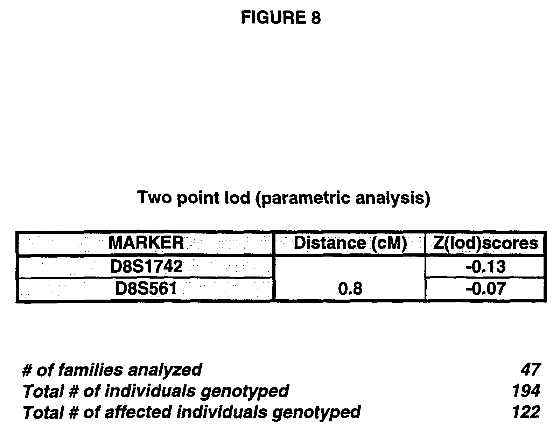

Figure 8 shows the results of a linkage analysis on 194 individuals issued from 47 families affected by prostate cancer. Two point lod score parametric analysis was performed using two microsatellite markers flanking the region of chromosome 8 defined in Figure 1. Lod scores obtained suggest the absence of any linkage between prostate cancer and loci within the region.

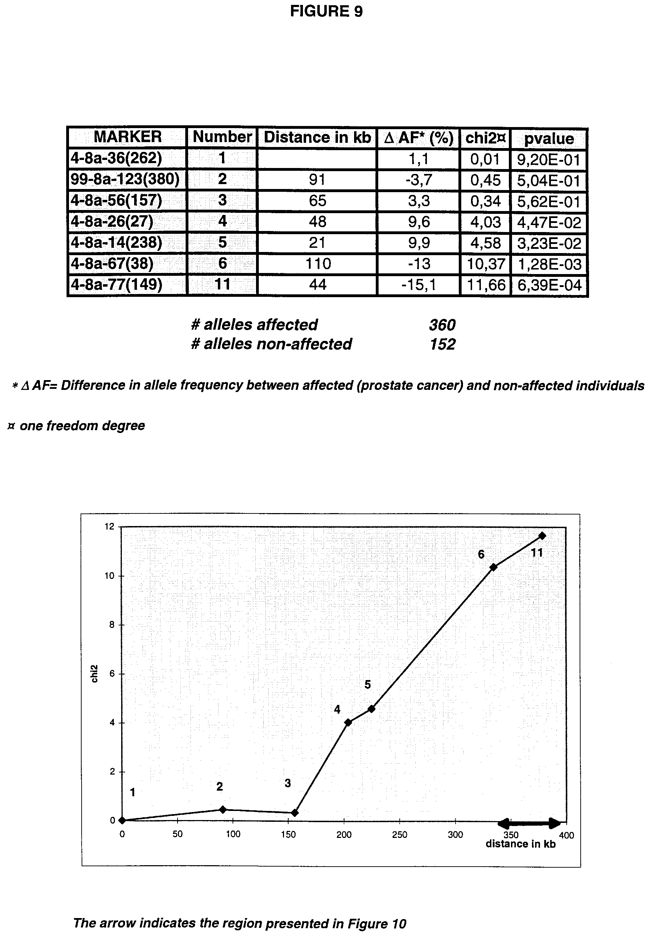

Figure 9 illustrates the identification of a candidate region associated with prostate cancer in the 8p23 chromosomal segment. The markers described in Figure 1 were individually genotyped as in Figure 6, in 180 prostate cancer patients and 77 non affected controls. Allelic frequencies were calculated in the affected and the non affected populations. For each marker, ΔAF represents the difference of allelic frequencies between the two populations. Significance of DAF was assessed by calculating $X^2$ (one degree of freedom) and p-values. The graph presents $X^2$ values for the whole set of markers positioned along the chromosomal locus (distances are expressed in kilobases).

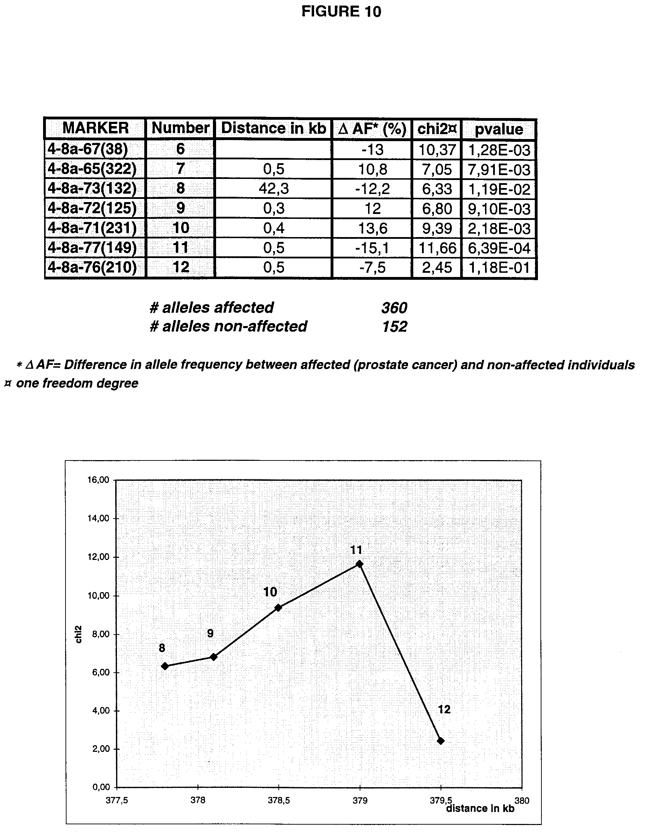

Figure 10 presents a similar experiment as that of Figure 9, with new markers generated at a higher density, around those showing the highest ΔAF values.

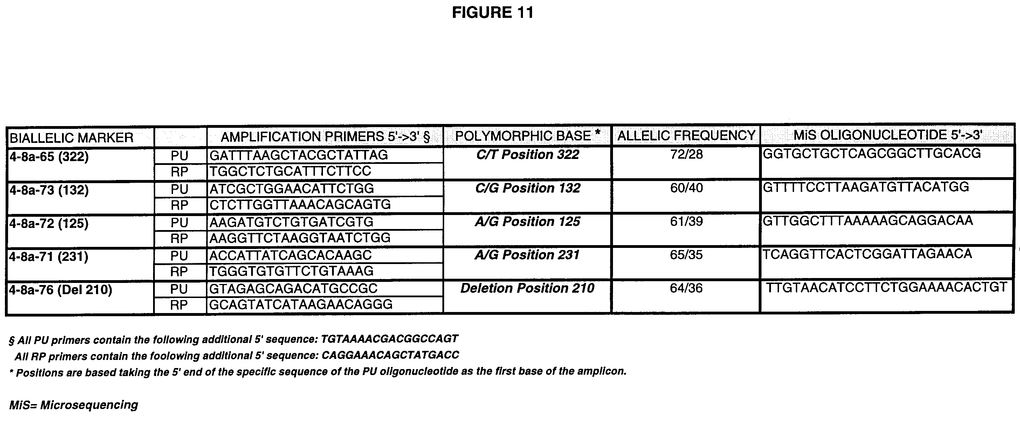

Figure 11 describes the oligonucleotides used to generate and genotype the markers of Figure 10.

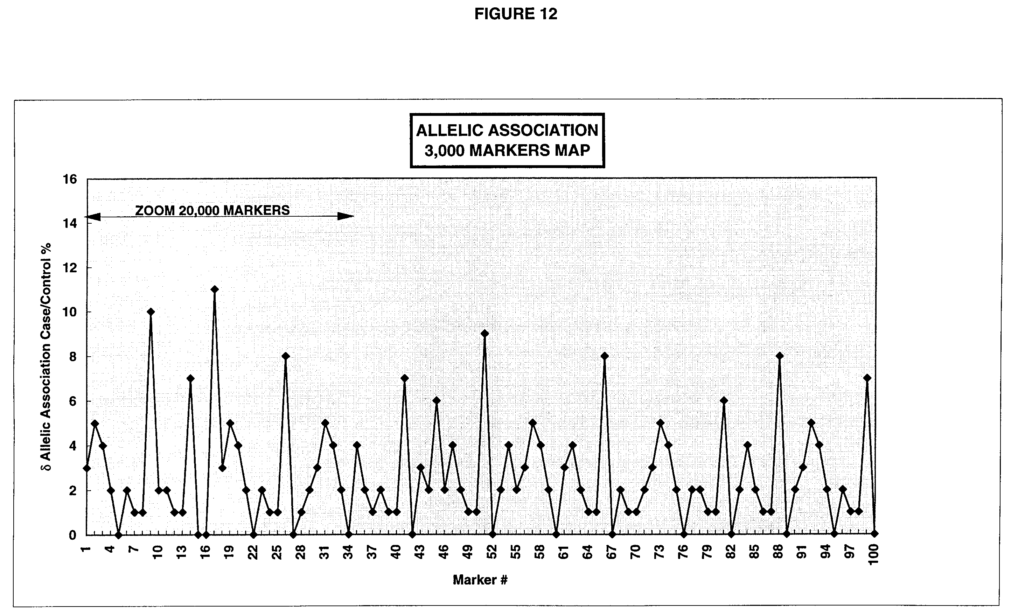

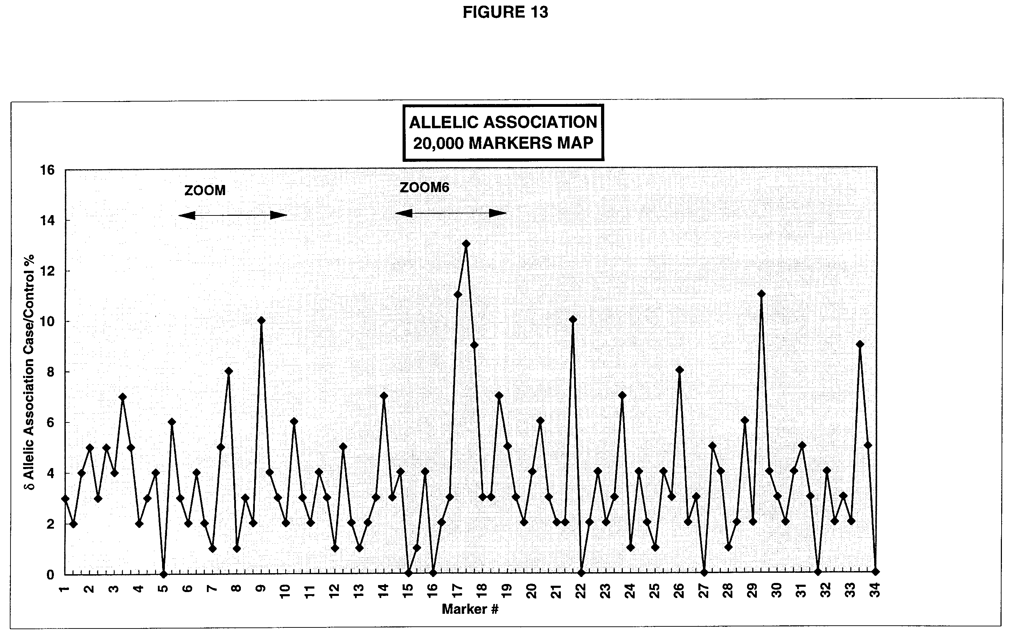

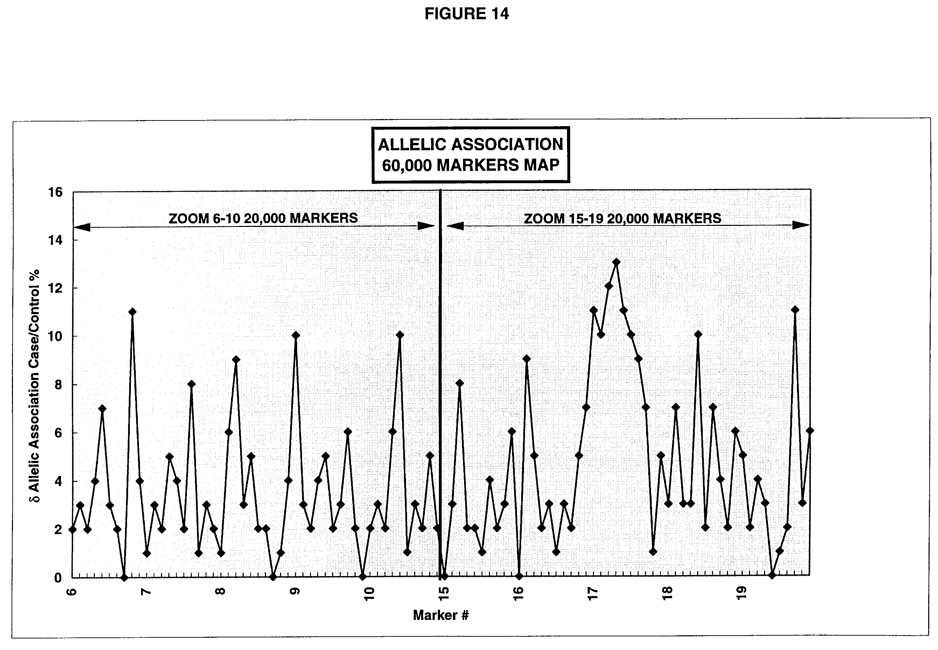

Figures 12, 13 and 14 illustrate the increasing reliability of association studies with the stepwise generation of bi-allelic marker maps of increasing densities, based on a statistical analysis of numerous random value samples.

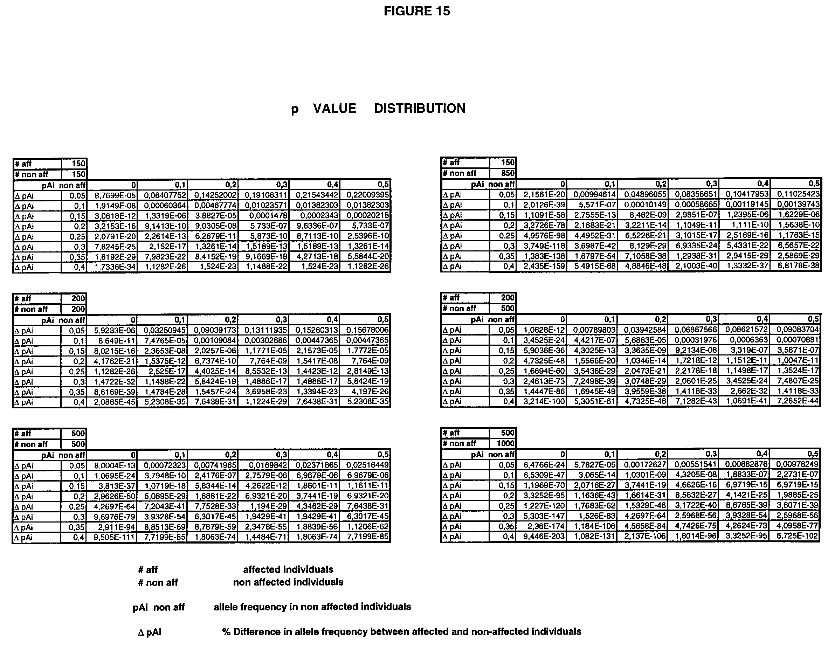

Figure 15 establishes the significance of association studies as a function of the size of trait + and trait - samples, and the frequency of the studied allele in the population.

<u>Methods used for the generation and utilisation of the high density bi-allelic marker map</u>

<u>Materials and Methods</u>

The generation of the invention's high density bi-allelic marker map results from the co-ordinated interaction of five fully integrated, industrial scale, methods: oligonucleotide synthesis, high throughput BAC libraries mapping and subcloning, high throughput sequencing, bioinformatics analysis and genomics analysis, including automated microtiter plate microsequencing.

a) Oligonucleotide synthesis

Oligonucleotide primers are synthesized on patented GENSET UFPS 24.1 Ultra Fast Parallel Synthesizers using phosphoramidite chemistry applied to a universal support (Ref brevets).

b) DNA extraction

Genomic DNA is extracted from blood samples (20 ml peripheral blood) obtained from appropriate healthy individuals using a standard procedure (Sambrook J, Fritsch EF, Maniatis T, 1989).

c) Genomic PCR

-   Oligonucleotide primers for genomic PCR amplification are designed using the OSP computer software (Hillier et al. , 1991).
-   Couples of oligonucleotide primers are designed in order to amplify the sequences derived from every ordered BAC. All primers contain, upstream of the specific target bases, a common oligonucleotide tail for sequencing (PU : TGTAAAACGACGGCCAGT, for the forward primers ; RP : CAGGAAACAGCTATGACC, for the reverse primers).

Amplification of each BAC-derived sequence is carried out using the polymerase chain reaction under the following conditions :

| Final volume | 50 µl |
|---|---|
| Genomic DNA | 100 ng |
| MgCl2 | 2 mM |
| dNTP (each) | 200 µM |
| Primer (each) | 7.5 pmoles |
| AmpliTaq Gold DNA polymerase | 1 unit |
| PCR buffer | 1 X |
| (10 X corresponds to 0.1 M Tris HCl pH 8.3, 0.5 M KCl) | |

Samples are subjected to 35 amplification cycles of 94°C for 30 sec, 55°C for 1 min and 72°C for 30 sec, followed by a final elongation step for 7 min at 72°C, in an appropriate thermocycler.

Amplification products are quantified in 96-well plates using the double-stranded DNA-specific dye Picogreen (Molecular Probes) and a microtiter fluorometer.

d) Detection of a microsequencing reaction on microtiter plates

The detection of a microsequencing reaction by a solid phase assay lies on the use of 5'-biotinylated oligonucleotides and fluorescein-dideoxynucleotides (DUPONT NEN). The assay is entirely carried out in a microtiter plate format. The biotinylated oligonucleotide anneals to the target nucleic acid immediately adjacent to the polymorphic nucleotide position of interest. Once specifically extended at the 3' end by a DNA polymerase using the complementary labelled dideoxynucleotide analog (PCR cycle), the biotinylated primer is captured on a microtiter plate coated with streptavidin (BOEHRINGER). The incorporated ddNTP is detected by a fluorescein antibody - alcaline phosphatase conjugate (BOEHRINGER).

20 µl of the microsequencing reaction added to 80 µl of capture buffer (SSC 2X, 2.5% PEG 8000, 0.25 M Tris pH7.5, 1.8% BSA, 0.05% Tween 20) are incubated for 20 minutes on a microtiter plate coated with streptavidin (Boehringer). Then the plate is rinsed once with washing buffer (0.1 M Tris pH 7.5, 0.1 M NaCl, 0.1% Tween 20), and 100 µl of anti-fluorescein antibody diluted 1/5000 in washing buffer containing 1.8% BSA are incubated in the microtiter plate for 20 minutes. After washing four times the microtiter plate, 100 µl of 4-methylumbelliferyl phosphate (Sigma) diluted to 0.4 mg/ml in 0.1 M diethanolamine pH 9.6, 10mM $MgCl_2$ are added. The detection of the microsequencing reaction is carried out on a fluorimeter (Dynatech) after 20 minutes of incubation.

e) High Throughput Sequencing

High Throughput Sequencing is performed on thirty automated ABI 377 sequencers, together with five ABI turbocatalysts robots performing the sequencing reactions. PCR is conducted on Perkin thermocyclers, Biomek and Hamilton robots are used for mix and sample preparation. A staff of 33 skilled technicians in two-shifts operation, routinely performs PCRs, sequencing reactions, gel preparation, and gel electrophoresis on ABI 377.

Amplification products from genomic PCR are subjected to automated dideoxy terminator sequencing reactions using Thermosequenase DNA polymerase and a dye-primer cycle sequencing protocole (ABI fluorophores). Sequencing reactions are assembled essentially as described by the manufacturer (Amersham). Reactions are conducted in a 96-well format using an appropriate thermal cycler. Temperature profiles are as follows. For PU dye-primer sequencing reactions: 95°C, 4 sec ; 55°C, 10 sec ; 70°C, 1 min (15 cycles) followed by 15 cycles of 95°C, 4 sec ; 70°C, 1 min. For RP dye-primer sequencing reactions, the profile is the same, except that the annealing temperature is 50°C.

After thermal cycling, sequencing reactions are ethanol precipitated, resuspended in loading buffer containing formamide, denatured, and electrophoresed on ABI 377 sequencing machines.

Two informatic networks and in-house developed software are in charge of the real-time controlling and sample tracking during the production process, and the automatic quality control and validation of the sequences. This software runs on UNIX platforms.

The quality control and validation software has two main functions. First, it makes a reassignment of bases, and corrects errors in the base-calling that were done by the ABI base-caller. Second, and more important, it automatically calculates, with very stringent criteria, confidence values for stretches of bases in the sequencing profiles that are generated by the sequencers. This allows to automatically and accurately cut-off and eliminate sequences or stretches of bases for which the confidence value would be less than 99%. This guarantees very efficiently the quality of the data that are finally transferred to the central database.

For sequence assembly, public domain software is used, such as the XGAP/XBAP package as well as in-house developed software to allow quick and accurate contigation process of the sub-BAC fragments.

f) Bioinformatics analysis

Since genes and regulatory regions are scattered throughout the genome, but make up only about 5% of the genome, special techniques must be used to find them.

Once a region has been sequenced, several complementary techniques will be used to detect genes and regulatory regions.

In parallel to the sequence assembly process, each BAC fragment (~500bp) goes through an automatic software analysis, including the following set of well known procedures: locating repeats, retaining the "informative" sequence, and checking it against numerous databases, looking for highly probable exons by using a set of scoring algorithms (such as trained Hidden Markov Models, statistical analysis models, including promoter-prediction tools, and the GRAIL neural network).

Preferred databases include:

- NetGene database:

This proprietary database contain sequences of 5' cDNA tags, obtained from a number of tissues and cells. Currently more than 45,000 different 5' clones representing more than 45,000 different genes are included in NetGene. The sequences in the NetGene database correspond specifically to the 5' regions of transcripts (first exons) and therefore allow mapping of the beginning of genes within raw genomic sequences.

- NRPU (Non-Redundant Protein-Unique) database:

Which is a non- redundant merge of the publicly available NBRF/PIR, Genpept, SwissProt databases. Homologies found with NRPU allow the identification of regions potentially coding for already known proteins or related to known proteins (translated exons).

- NREST (Non-Redundant EST database):

Merge of the EST subsection of the publicly available GenBank database. Homologies found with NREST allow the location of potentially transcribed regions (translated or non-translated exons).

- NRN (Non-Redundant Nucleic acid database):

Merge of GenBank, EMBL and their daily updates. Homologies found with NRN have to be manually checked.

Any sequence giving a positive hit with NRPU, NREST or an "excellent" score with GRAIL or/and other scoring algorithms is considered a potential functional region (exon or promoter), and is then considered a candidate for genomic analysis.

While this first screening allows the detection of the strongest exons, a semi- automatic scan is further applied to the remaining sequences in the context of the sequence assembly. That is, the sequences neighbouring a 5' site or an exon in a subBAC are submitted to another round of bioinformatics analysis with modified parameters. New exon candidates are thus generated for genomic analysis.

Map characteristics

The map described in the invention is composed of a set of bi-allelic markers having the following characteristics:

- high density : it comprises over 20,000 markers;
- polymorphic informative content of markers : each marker has a heterozygosity rate higher than about 42%;
- homogeneous : the markers are evenly distributed along the genome, with an average inter-marker spacing lower than 150 kilobases. Furthermore, linkage disequilibrium regions are taken into account in order to select an optimal set of markers, as described further.

Generation of the Map

The generation of the high density bi-allelic marker map involves the following steps :

- Generation of a human genomic DNA library of high quality cloned in an appropriate vector (100 to 300 kilobases inserts, non-chimeric, sequence-ready).In a preferred embodiment, BACs are used as vectors of choice, and insert fragments have a 100-200 kb length.
- Construction of a physical map with 10,000 to 20,000 minimally overlapping ordered clones. In the above mentioned embodiment, 15,000 to 20,000 BACs are ordered in order to constitute a minimally overlapping set covering the entire human genome.
- Partial sequencing of the selected ordered clones.
- Generation of several bi-allelic markers per at least partially sequenced clone or BAC insert.

**Example 1: Generation of a human genomic DNA library**

Physical maps consist of ordered, overlapping cloned fragments of genomic DNA covering each chromosome. Physical mapping in complex genomes such as the human genome (3,000 Megabases) requires the construction of DNA libraries containing large inserts (in the order of 0.1 to 1 Megabase). It is crucial that such libraries be easy to construct, screen and manipulate, and that the DNA inserts be stable and relatively free of chimerism. Yeast artificial chromosomes (YACs ; Burke et al. 1987) have provided an invaluable tool in the analysis of complex genomes since their cloning capacity is extremely high (several Mb). YAC libraries containing large DNA inserts (up to 2 Mb) have been used to generate STS-content maps of individual chromosomes or of the entire human genome (Chumakov et al. 1992; Chumakov et al. 1995; Gemmill et al. 1995; Doggett et al. 1995 ; Hudson et al. 1995). Even though YACs have been crucial tools for the assembly of physical map frameworks of the human genome, as well as for cloning disease genes based on their chromosomal position (positional cloning projects), the reliability of YACs for mapping and sequencing purposes is often limited by problems such as a high rate of chimerism (40 to 50% of clones containing fragments from more than one genomic region), the clonal instability of some regions, and a tedious procedure to manipulate and isolate YAC insert DNA. Therefore, in order to generate an integrated physical and genetic map such as that required for the purpose described in this patent, one has to construct a genomic DNA library in a system which retains the advantages of enabling large insert size cloning and yet remaining stable, of being easy to manipulate, and of allowing standard implementation of molecular biology techniques.

The bacterial artificial chromosome (BAC) cloning system (Shizuya et al.) is capable of stably propagating and maintaining relatively large genomic DNA fragments (up to 300 kb long) as single-copy plasmids in *E.coli*. BACs are further characterised by a low rate of chimerism and fragment rearrangement, together with a relative ease of insert isolation. Thus BAC libraries are well suited to integrate genetic, STS and cytogenetic information while providing direct access to stable, sequence-ready human DNA.

Any other type of vector presenting at least similar properties as BACs will also be suitable to generate the map according to the invention.

Human genomic BAC libraries

Human genomic BAC libraries were obtained as described in Woo et al., 1994. Briefly, two different whole human genome libraries were produced by cloning partially digested DNA from a lymphoblastoid cell line (derived from individual N° 8445, CEPH families) into pBeloBAC11 vector (Kim et al. 1996). The library produced with BamHI partial digestion contains 110,000 clones with an average insert size of 150 kb, that corresponds to 5 human haploid genome equivalents. The library prepared with HindIII enzyme corresponds to 3 human genome equivalents with 150 kb average insert size of the clones. DNA from the clones of both libraries was isolated and pooled in a three dimensional format ready for PCR screening (see below).

**Example 2: Construction of a physical map**

In order to generate the high density bi-allelic marker map, 15,000 to 20,000 BACs are physically ordered by screening the above described BAC libraries with ca. 20,000 STS markers. Such screening is implemented until one positive BAC clone per STS is isolated, thus generating a minimally overlapping set of 15,000 to 20,000 BACs covering the whole human genome.

BAC screening

Three-dimensional pools of the total human DNA libraries are screened for 20,000 ordered STS amplification, by high throughput PCR methods (Chumakov et al. 1995). Briefly, three dimensional pooling consists in rearranging the (thousands of) samples to be tested in a manner which allows to reduce the number of reactions required by at least 100 fold, as compared to screening each clone individually. Positive bands generated are detected by conventional agarose gel electrophoresis combined with automatic image capturing and processing. In a final step, STS-positive clones are checked individually. Subchromosomal localisation of BACs is systematically verified by fluorescence in situ hybridisation (FISH), performed on metaphasic chromosomes as described by Cherif et al 1990. BAC insert sizing is determined by Pulsed Field Gel Electrophoresis after digestion with restriction enzyme NotI.

**Example 3: Partial sequencing of BAC clones**

The ordered BACs selected by STS screening and verified by FISH, are partially sequenced using the following process, with standard laboratory protocols.

BAC subcloning

Each BAC human DNA is first extracted using the alkaline lysis procedure and then sheared by sonication. The obtained DNA fragments are end-repaired and electrophoresed on a preparative agarose gel. The fragments in the size range from 600 to 1,000 bp are isolated from the gel, purified and ligated to a linearised, dephosphorylated, blunt-ended plasmid cloning vector (pBluescript II Sk (+)).

Partial sequencing of BACs

The ligated products are electroporated in the appropriate cells (ElectroMAX *E.coli* DH10B cells). IPTG and X-gal are added to the cell mixture, which is then spread on the surface of an ampicillin-containing agar plate. After 37°C overnight incubation, recombinant (white) colonies are randomly picked and arrayed in 96 wells microplates. At least 30 of the obtained subBAC clones are sequenced by the end pairwise method (500 bp sequence from each end) using a dye-primer cycle sequencing procedure as described in Materials and Methods. Pairwise sequencing is performed until a map allowing the relative positioning of selected markers along the corresponding DNA region is established.

**Example 4: Generation of bi-allelic markers**

As shown in the following results (《《 Distribution of informative bi-allelic polymorphisms in the human genome 》》), the frequency of the bi-allelic polymorphisms used to construct the high density marker map (bi-allelic polymorphisms with a heterozygosity rate higher than 42%) is one in 2.5 to 3 kb. Therefore, six 500 bp-genomic fragments have to be screened in order to derive 1 bi-allelic marker. Six pairs of primers, each one defining a 500 bp amplication fragment, are derived from the above mentioned BAC partial sequences. All primers contain, upstream of the specific target bases, a common oligonucleotide tail for sequencing. Amplification of each BAC-derived sequence is carried out on pools of DNA from 100 individuals. The conditions used for the polymerase chain reaction have been optimised so as to obtain more than 95% of PCR products giving 500bp-sequence reads.
Amplification products from genomic PCR (further described in Materials and Methods) are subjected to automated dideoxy terminator sequencing reactions using a dye-primer cycle sequencing protocole. Following gel image analysis and DNA sequence extraction, sequence data are automatically processed with adequate software to assess sequence quality and to detect the presence of bi-allelic sites among the pooled amplified fragments. Bi-allelic sites are systematically verified by comparing the sequences of both strands of each pool. Further details on sequencing and bioinformatics procedures are provided in Materials and Methods.
The detection limit for the frequency of bi-allelic polymorphisms detected by sequencing pools of 100 individuals is 0.3 +/- 0.05 for the minor allele, as verified by sequencing pools of known allelic frequencies. Thus, the bi-allelic markers selected by this method will have a frequency of 0.3 to 0.5 for the minor allele and 0.5 to 0.7 for the major allele, thus a heterozygosity rate higher than 42%.

Results

a) Distribution of informative bi-allelic polymorphisms in the human genome

In order to estimate the average distribution of bi-allelic markers presenting a high informative content (heterozygosity rate higher than about 42%), 300 different amplicons derived from 100 individuals, and covering a total of 150 kb issued from different genomic regions, were sequenced. A total of 54 such informative bi-allelic polymorphisms were identified, which shows that there is one bi-allelic polymorphism with an heterozygosity rate higher than 42% every 2.5 to 3 kb. Given the human genome is $3.10^6$ kb long, this indicates that, out of the $10^7$ bi-allelic markers present on the human genome, $10^6$ would be suitable for genetic mapping purposes.

b) Generation of seven bi-allelic markers spanning over a 550 kb region of chromosome 8.

Figure 1 shows the distribution of seven bi-allelic markers interspaced by 20-110 kb, and and average inter-marker distance of ca. 60 kb.
Figure 2 shows the oligonucleotides used to generate such a fragment of the high density bi-allelic marker map.

In a preferred embodiment of the invention, an intermediate map of ca. 20,000 markers (1 marker per BAC) is generated, and another preferred embodiment of the invention is a final map of 60,000 markers (3 markers per BAC).
Figure 3 shows the results of a computer simulation establishing the preferred numbers of markers to be generated per BAC, depending on the targeted average inter-marker spacing. It shows that :

- 98% of inter-marker distances will be lower than 150kb provided 60,000 evenly distributed markers are generated (3 per BAC)

- 90% of inter-marker distances will be lower than 150kb provided 40,000 evenly distributed markers are generated (2 per BAC)
- 50% of inter-marker distances will be lower than 150kb provided 20,000 evenly distributed markers are generated (1 per BAC).

Utilisation of the Map

The routine, industrial scale usage of the high density map requires cost- and time-effective, reliable, routine genotyping techniques. Genotyping large populations by means of sequential pooling procedures allows to reduce the number of tests to be achieved to analyse all markers in a population. Furthermore, the invention presents the use of refined microsequencing techniques, based on either gel electrophoresis or microtiter plate analysis, as best enabling methods to conduct high throughput genotyping.

**Example 5: High Throughput Genotyping of bi-allelic markers by Microsequencing**

Genotyping of bi-allelic markers is determined by performing microsequencing reactions on amplified fragments obtained by genomic PCR, in similar conditions to those used for the generation of bi-allelic markers. Microsequencing reactions can be equally performed on individual or pooled DNA samples. After amplification of the fragment to be tested, unincorporated dNTPs are eliminated by incubation with shrimp alkaline phosphatase and exonuclease I, according to manufacturer's recommendations.

Amplification products from genomic PCR are subjected to automated microsequencing reactions using fluorescent ddNTPs and the appropriate oligonucleotide primer, which hybridises just upstream of the polymorphic base. After thermal cycling, microsequencing reactions are analyzed either by electrophoresis on ABI 377 sequencing machines or by a solid phase microtiter plate assay. Details of the microtiter plate assay are provided in Materials and Methods.

Following gel image or fluorimeter analysis, data are automatically processed with a software which allows to determine either the individual genotypes or the allele frequencies of bi-allelic markers within the pooled amplified fragments.

The detection limit for the frequency of bi-allelic polymorphisms detected by microsequencing pooled DNA samples is 0.2 +/- 0.05 for the minor allele, as verified by sequencing pools of known allelic frequencies.

Association studies using the high density bi-allelic marker map

Linkage Disequilibrium Regions

If two genetic loci lie on the same chromosome, then sets of alleles on the same chromosomal segment (*i.e.* haplotypes) tend to be transmitted as a block from generation to generation. When not broken up by recombination, haplotypes can be tracked not only through pedigrees but also through populations. The resulting phenomenon at the population level is that the occurrence of pairs of specific alleles at different loci on the same chromosome is not random, and the deviation from random is called linkage disequilibrium.

Linkage disequilibrium between two alleles is primarily determined by the recombination frequency between the alleles loci. In most cases, the recombination frequency only depends on the distance between the two loci: recombination will rarely separate loci which lie very close together on a chromosome, while the further apart two loci are on a chromosome, the more likely it is that a crossover will separate them.

By definition, two loci which show a 1% recombination rate per meiosis are defined as being 1 cM apart on a genetic map. Equivalence of genetic distance and physical distance based on chiasma counts has been estimated as 1cM = 0.9 Mb (sex-average ; 1.13 Mb in males and 0.67 Mb in females). However, the actual correspondence between genetic and physical distances varies widely for different chromosomal regions due to the presence of recombinational hot spots.

It has been anticipated that bi-allelic markers within regions between recombination hot spots are usually in linkage disequilibrium. This is depicted in the following scheme :

```
      LDR 1      LDR2        LDR 3           LDR 4         LDR 5    LDR 6    LDR7
   X            X          X                X            X        X      X
```

X    Putative Recombination hot spot

LDR    Linkage Disequilibrium Region

Example 6 illustrates this concept by measuring the linkage disequilibrium (LD) between bi-allelic markers derived from BACs.

## Example 6: Identification of a putative recombinational hot spot

LD among a set of bi-allelic markers having a heterozygosity rate of ca. 50%. was determined by genotyping 100 unrelated individuals corresponding to a heterogeneous population constituted of random blood donors collected at several hospitals in Paris. Genotyping was performed through individual microsequencing reactions.

LD between two bi-allelic markers $(M_i, M_j)$ was calculated for every allele combination $(M_{i1}, M_{j1}$ ; $M_{i1}, M_{j2}$ ; $M_{i2}, M_{j1}$ and $M_{i2}, Mj_2)$, according to the Piazza formula :

$$\Delta M_{ik}, M_{jl} = \sqrt{\theta 4} - \sqrt{(\theta 4 + \theta 3)(\theta 4 + \theta 2)} ,$$

where :

$\theta 4 = - - =$     frequency of genotypes not having allele k at $M_i$ and not having allele l at $M_j$
$\theta 3 = - + =$     frequency of genotypes not having allele k at $M_i$ and having allele l at $M_j$
$\theta 2 = + - =$     frequency of genotypes having allele k at $M_i$ and not having allele l at $M_j$

Results: identification of a putative recombinational hot spot in genomic region 1q21

Figure 4 shows a putative recombination hot spot between 2 markers separated by 37kb on chromosome 1q21. Figure 5 describes the oligonucleotides used to generate these results.

Trait localisation on Linkage Disequilibrium Regions

considering a genetic trait, the trait locus will be in LD with flanking markers situated in the same linkage disequilibrium region (LDR), as schematised below:

```
            Trait locus
                |
      LDR 1     |     LDR2        LDR 3           LDR 4         LDR 5    LDR 6    LDR7
   X            X          X                X            X        X      X
```

X    Putative Recombination hot spot

LDR    Linkage Disequilibrium Region

Therefore, specific alleles of these flanking markers must be found associated to the trait.

This situation is illustrated by the case of late onset Alzheimer's Disease (AD) and Apo E, as depicted in the following scheme :

This LD map is based on the data reported by Mullan *et al.*. 1996, for the Apo E/Apo CI loci, and data by Houlston *et al.*. 1989, for Apo E/Apo CII.

The allelic frequencies for Apo E and Apo CI alleles in a population-based sample (Florida, USA) are as follows :

| Allele | AD | Unaffected |
|---|---|---|
| Apo E e4 | 0.32 | 0.15 |
| Non-Apo E e4 | 0.68 | 0.85 |
| Apo CI H2 | 0.36 | 0.22 |
| Non-Apo CI H2 | 0.64 | 0.78 |

indicating a clear association between AD, and Apo E e4 (Relative Risk = RR = 2.7) or Apo CI H2 (RR = 2.0) alleles.

On the contrary, there is no significant association between AD and any Apo CII allele, which is located very closely to Apo E, thus suggesting the presence of a recombination hot spot between the Apo CII and Apo E loci.

Thus, the optimal genetic map to use efficiently the basic linkage disequilibrium property depends on the genome-wide distribution of recombinational hot spots.

### Use of LDRs to minimise the number of necessary markers to compose the high density map of the invention

Another preferred embodiment of the invention is to check for linkage disequilibrium pairs of genetic markers generated at each step of the map's elaboration, and to generate further markers in any region where no linkage disequilibrium has been demonstrated. This approach allows to minimise the number of markers to be generated, and to refine the map in regions were the recombination rate reveals higher than average.

The possibility to adjust the density of a genetic map in order to take LDRs into account, depends on the average size and distribution of LDRs along the human genome. Given a population founded recently i.e. a few centuries ago, by a few individuals, which did not mix with other populations, and given two adjacent loci with a founder's haplotype ab, a recombination event could separate a from b at each meiosis. Therefore the chance that a and b remain on the same haploid genome diminishes from generation to generation. In principle, the smaller the A-B distance, the more generations are required to eliminate the LD. This phenomenon is called LD by recent founder effect. In such populations (e.g. French Canadian), LD can be detected between several loci spanning rather large regions of the genome (one to several Megabases). However, in heterogeneous populations with various ancestral founders, LD has sometimes been analysed, and described along regions of several Megabases, as in the case of the HLA region.

To better estimate LDRs size and distribution, bi-allelic markers were generated in several random regions of 100 to 150 kb, and tested for LD in a French heterogeneous population.

**Example 7: Linkage disequilibrium region on chromosome 8**

Linkage disequilibrium was measured in the above mentioned French population for each pair of the bi-allelic markers generated in Example 4, using a software implementing the Piazza formula approach.

The resulting LD matrix presented in Figure 6 suggests the existence of two recombination hot spots between pairs of markers. Therefore, a corresponding LDR would span over ca. 100- 150 kb between these two hot spots. Figure 7 shows the oligonucleotides used to genotype the set of markers using the microsequencing technique.

This study indicated that the genomes from such a population very often comprise bins of adjacent polymorphisms in LD spanning 100 to 150 kb, with no or weak evidence for LD between alleles from adjacent bins. Within these bins the LD strength is not always correlated with the physical distance separating the markers or even sometimes not correlated with their order.

Assuming a majority of LDRs are 100 to 150 kb long, there are about 20 to 30,000 LDRs in the human genome.

As mentioned before, the mean distance between bi-allelic markers constituting the high density map will be less than 150 kb. With a 20,000 - 60,000 marker set having a uniform density, it can be estimated that most LDRs will be covered by at least one marker, assuming that the average distance between recombinational hot spots is in the range of 100-150 kb (total number of LDRs = 20,000-30,000). The lower the number of hot spots, the higher the coverage of LDRs by the high density marker map.

With a set of 60,000 markers, the majority of LDRs will be covered by several markers that will be in strong but unequal LD. In these bins, haplotypes of several alleles can be determined in order to enhance the statistical power of the association studies.

<u>High density map, Linkage Disequilibrium Regions and Association studies</u>

Association studies using the map described in the invention will allow to observe population association between allele A at a Marker locus and Trait T due to four reasons :

1) Allele A can directly cause susceptibility to T (*eg*, Apo E e4 allele and Alzheimer's disease). Since the majority of the bi-allelic markers are selected randomly, they mainly map outside genes. The likelihood of allele A being a functional mutation directly related to trait T is therefore very low.

2) The Marker locus is very closely linked to the trait locus : allele A is in linkage disequilibrium with the trait-causing allele. Then, a gene should be discovered near the Marker locus, which carries mutations in people with trait T. Moreover, if a high density marker map is used so that several markers are found in the same LDR, then the location of the causal gene can be deduced from the profile of the association curve : the causal gene will be found in the vicinity of the marker showing the highest association (*eg* AD for Apo C1 H2 RR = 2.0, while for the causal Apo E e4 RR = 2.7). This is the rationale for the use of the invention.

**Example 8: Candidate association peak on chromosome 8.**

Chromosomal region 8p23 is suspected of being involved in numerous pathologies, especially cancers: examples of documented associations with 8p23 region include hepatocarcinoma (Becker et al. 1996), non small cell lung cancer (Sundareshan et Augustus 1996,), prostate cancer (Ichikawa et al. 1996), and colorectal cancer (Yaremko et al. Genes 1994).

While these results were generated mostly by showing loss of heterozygosity (LOH) in the region, linkage analyses conducted on patients from prostate cancer affected families did not allow to locate candidate genes within the suspected region. The results of such an analysis are shown in Figure 8. In order to identify putative susceptibility genes associated with prostate cancer in the region of interest, we conducted association studies using the fragment of high density marker map presented in Figure 1. Results are shown in Figure 9, and reveal a candidate association region spanning over 50-100 kb. As already mentioned, a preferred embodiment of the invention consists in confirming the putative association by generating more markers within the candidate region. Figure 10 shows the results of such an experiment. The oligonucleotides used to generate this refined analysis are described in Figure 11.

3) People with the trait and people without the trait may be genetically different subsets of the population, who coincidentally also differ in the frequency of allele A (population stratification). This phenomenon may be balanced when using large heterogeneous samples.

4) Association between allele A and the trait is false and only results from sampling error, a phenomenon which is classically considered as increasing as a function of the number of markers tested.

a) The use of a high density map allows to highlight the causal associations, since the coincidental associations will be randomly distributed over the map, while the real associations will map in the same regions, giving rise to peaks compared to unique points.

**Example 9**

A simulation of such a situation is shown in Figures 12, 13, 14. This example shows the interest of refining the map in regions where initial association is found using a low density map, in order to identify true candidate association loci.

b) Statistical significance evaluation of candidate associations should take into account the total number of LDRs in the genome. If one is testing 60,000 markers, and assuming 25,000 LDRs, any significant p value (lower than $10^{-2}$) should theoretically be divided by $2.5 \times 10^4$ when testing allelic association, and by $6.25 \times 10^8$ when testing allelic interaction. In such a case, a conservative statistical interpretation implies considering an association as positive when its p value is lower than $4 \times 10^{-5}$, and considering an interaction as positive when its p value is lower than $1.6 \times 10^{-11}$.

**Example 10**

Figure 15 establishes the sample sizes required in order to obtain significant results from association studies performed on the high-density bi-allelic marker map, according to the p-value criteria defined above. Depending on the relative risk tested, samples ranging from 150 to 500 individuals are numerous enough to achieve statistical significance.

This method is thus particularly suited to the efficient identification of susceptibility genes which present common polymorphisms, and are involved in multifactorial traits whose frequency is relatively higher than that of diseases with monofactorial inheritance. Particular instances of such genes include the so far identified ApoE ; HLA DR; HLA B; ACE ; AGT.

Applications of the High Density Linkage Disequilibrium based Map

a) Association studies and the analysis of a disease

The general strategy to perform the association studies using the high density map, is to scan two pools of individuals (diseased patients and non-diseased controls) characterised by a well defined phenotype in order to measure the allele frequencies of more than 20,000 bi-allelic markers in each of these pools.

Allele frequency is measured using the microsequencing technique. Since two pools are being compared, the total number of allele frequency measurements that are performed in the association studies will be twice the number of markers used in the study.

An important embodiment of the invention is to set-up an on-line process between the generation of the bi-allelic markers and the corresponding analysis of their frequency in the different pools. Using this particular embodiment, it is not necessary to have completed a full high density bi-allelic marker map in order to start the association study. It is sufficient to generate a first set of at least ca. 20,000 markers (one marker per BAC) and to simultaneously conduct the association study. The rest of the high density marker map (comprising up to two more markers per BAC) is then generated by starting first on those BACs for which a candidate association has been estblished at the first step.

Even when the full high density bi-allelic marker map (ca. 60,000 markers) is available, it is not necessary to use the whole map in order to start an association study. It is sufficient to conduct a first step association study on an initial set of ca. 20,000 markers. More markers are then tested, priority being given to those BACs for which a candidate association has been established at the first step.

b) Association studies and the analysis of drug response : pharmacogenomics

An important use of the invention is the study of drug response.

Drug efficacy and tolerance/toxicity can be considered as multifactorial traits involving a genetic component in the same way as are complex diseases such as Alzheimer's Disease, hypertension or diabetes. As such, the identification of genes involved in drug efficacy and toxicity could be achieved following a positional cloning approach, e.g. performing linkage analysis within families in order to obtain the subchromosomal location of the gene(s). However, this type of analysis is actually impractical in the case of drug responsiveness, due to the lack of availability of familial cases. In fact, the likelihood of having more than one individual in a particular family being exposed to the same drug at the same time, is very low. Therefore, drug efficacy and toxicity can only be analysed as sporadic traits.

In order to conduct association studies to analyse the individual response to a given drug in groups of patients affected with a disease, up to four pools are screened:

- Non-diseased or random controls,
- Diseased patients/drug responders,
- Diseased patients/drug non-responders,
- Diseased patients/drug side effects.

The final number and composition of the pools for each drug association study is defined according to the patients' phenotypic data. Allele frequency will be measured by using the microsequencing technique.

For each studied drug, the total number of allele frequency measurements which is performed in the association studies will be :

TOTAL TESTS/DRUG = NUMBER OF MARKERS X NUMBER OF POOLS

In the same way as described for the analysis of a disease, a multi-step genotyping process testing markers at increasing densities allows to minimise the number of measurements and to focus on regions exhibiting a candidate association.

c) Association studies and the analysis of other sporadic traits

The invention can further be utilised in order to analyse any trait.

d) Interaction studies and the analysis of a polygenic disease

The analysis of genetic interaction between alleles at unlinked loci requires individual genotyping. Allelic interaction among a selected set of bi-allelic markers with appropriate p-values can be studied as an association, provided the analysis is run on individual DNAs from different diseased sub-populations. Allelic typing can optimally be performed by using the microsequencing technique.

e) Gene identification

If a positive association with a disease, or with drug efficacy or toxicity is identified using the high density bi-allelic marker map, this map will provide not only the confirmation of the association, but also a short cut towards the identification of the gene involved in the trait under study. As mentioned below, since the markers showing positive association to the trait are in linkage disequilibrium with the trait loci, the causal gene will be physically located in the vicinity of these markers. Regions identified through association studies using the high density map will on average have a 20 - 40 times shorter length than those identified by linkage analysis (2 to 20 Mb).

Gene localisation

Once a positive association is confirmed with the high density bi-allelic marker map, BACs from which candidate markers were derived are completely sequenced and the mutations in the causal gene are identified by applying genomic analysis tools.

Once a region has been sequenced and analysed, the candidate functional regions (exons and promoters) are scanned for mutations by comparing the sequences of a selected number of controls and cases, using adequate software (Materials and Methods). Candidate mutations are further confirmed by screening a larger number of cases and controls with the microsequencing technique.

Mutation detection

The mutation detection procedure is similar to that for the bi-allelic site detection.

A pair of oligonucleotide primers are designed in order to amplify the sequences of every exon/promoter predicted region. Amplification of each predicted functional sequence is carried out on DNA samples from affected patients and non-affected controls using the polymerase chain reaction under the above described conditions. Amplification products from genomic PCR are subjected to automated dideoxy terminator sequencing reactions and electrophoresed on ABI 377 sequencers. Following gel image analysis and DNA sequence extraction, ABI sequence data are automatically analysed to detect the presence of sequence variations among affected cases and non affected controls. Sequences are systematically verified by comparing the sequences of both DNA strands of each individual.

Candidate polymorphisms are then verified by screening a larger population of cases and controls by means of the microsequencing technique in an individual test format. Polymorphisms are considered as candidate mutations when present in cases and controls at frequencies compatible with the expected association results.

References

Becker et al., *Cancer Res*. 1996, 56 (21): 5092-5097
Benham., F.K. et al., *Genomics*, 1989, **4**: 509-517.
Burke et al. *Science* , 1987, <u>236</u>, 806-812
Chee et al., *Science* 1996, **274**: 610-614.
Cherif et al. *P.N.A.S.USA* 1990, **87** : 6639-6643.
Chumakov et al., *Nature* 1992, **359**: 380-387.
Chumakov et al., *Nature* 1995, **377**: 175-298.
Cox, D.R. et al., *Science* 1990, **250**: 245-250.
Dib et al., *Nature* 1996, **380**: 152.
Doggett et al., *Nature* 1995, **377**, 335-365
Gemmill et al., *Nature* 1995, **377**, 299-319
Gyapay et al., Nature Genet. 1994, **7**: 246-339.
Hillier L. and Green P. *Methods Appl.,* 1991, 1: 124-8.
Houlston et al. *Hum.Genet*. 1989, **83**:364-368
Hudson et al. *Science* ,1995, **270**, 1945-1954
Ichikawa et al. *Prostate Suppl* 1996, **6**: 31-35
Kim et al. *Genomics* ,1996, **34**, 213-218.
Lander, E. *Science* 1996, 274: 536-539.
Lathrop M., *Curr Op Biotechnology* , 1993, **4**: 678-683.
Mullan et al., *Ann.N.Y.Acad.Sci*. 1996, **802**: 16-26.
Peterson et al. *Human Mol. Genet*. 1995, **4**: 887-894.
Risch, N. and Merikangas, K., *Science* 1996, **273**: 1516-1517.
Sambrook J, Fritsch EF, Maniatis T, 1989.
Schuler GD et al, *Science* 1996, **274**: 540-546.
Shizuya et al. *Proc. Natl.Acad.Sci.USA* ,1992, **89**, 8794-8797
Sundareshan et Augustus, *Cancer Genet Cytogenet*, 1996, **91** (1): 53-60.
Wang et al. Abstracts of papers presented on genome Mapping and sequencing.May 14-18, 1997. Cold Spring harbor laboratory. P 17.
Woo et al. *Nucleic Acids Res.,* 1994, **22**, 4922-4931
Yaremko et al. *Genes Chromosomes Cancer*, 1994, **10**(1): 1-6.

**Claims**

1. Method for generating a high density linkage disequilibrium map of the human genome, comprising the steps of:

   a)ordering a set of 10,000 to 20,000 cloned genomic fragments along the human genome, with average size ranging from 100 kb to 300 kb;
   b) generating several bi-allelic markers per fragment; and
   c) selecting one to three bi-allelic marker per fragment, with heterozygosity rate higher than 40%.

2. Method for generating a high density linkage disequilibrium map of the human genome, comprising the steps of:

   a) ordering a set of 15,000 to 20,000 BACs along the human genome, with average insert size ranging from 100 kb to 200 kb;
   b) generating several bi-allelic markers per BAC; and
   c) selecting one to three bi-allelic marker per BAC, with heterozygosity rate higher than 40%.

3. Method according to claim 1 or 2 where bi-allelic markers are preferably generated in any region with no evidence of linkage disequilibrium.

4. Method according to claim 1 or 2 where bi-allelic markers are preferably generated in any region with evidence for a positive association with a genetic trait.

**5.** Map of the human genome obtained by a method according to any one of claims 1 to 4.

**6.** Subset of markers derived from a map according to claim 5.

**7.** Bi-allelic marker obtained by a method according to any one of claims 1 to 4.

**8.** Method of identifying one or several bi-allelic markers associated with a trait, comprising the steps of:

a) scanning a set of markers according to claim 5 or 6 in trait $^+$ and trait $^-$ individuals; and
b) establishing a statistically significant association between one allele of the marker(s) and the trait.

**9.** Method of identifying a gene associated with a trait, comprising the steps of:

a) identifying one or several marker(s) using a method according to claim 8; and
b) establishing a statistically significant association between one or several allele(s) of a gene in the vicinity of the identified marker(s) and the trait.

**10.** Method according to claim 8 where said trait is a disease.

**11.** Method according to claim 9 where said trait is a disease.

**12.** Method according to claim 8 where said trait is a drug response.

**13.** Method according to claim 12 where said response is efficacy, toxicity and/or tolerance.

**14.** Method according to claim 9 where said trait is a drug response.

**15.** Method according to claim 14 where said response is efficacy, toxicity and/or tolerance.

**16.** Marker obtained by a method according to any one of claims 8, 10, 12 and 13.

**17.** Oligonucleotide probe comprising a sequence capable of specifically hybridising with one allele of a marker according to claim 16.

**18.** Oligonucleotide primer comprising a sequence capable of specifically detecting one allele of a marker according to claim 16.

**19.** High density oligonucleotide array comprising probes comprising sequences capable of selectively hybridising with specific alleles of a set of markers according to claims 5 and 6.

**20.** High density oligonucleotide array comprising primers comprising sequences capable of selectively detecting specific alleles of a set of markers according to claims 5 and 6.

**21.** Oligonucleotide probe or primer capable of hybridising specifically with the sequence of one marker's allele identified by a method according to any one of claims 9, 11, 14 and 15.

**22.** Diagnostic assay using an oligonucleotide probe or primer according to claim 17, 18 or 21.

**23.** Diagnostic assay according to claim 22, where said oligonucleotide probe or primer is immobilised on a solid support.

**24.** Gene associated with a trait which is identified by a method according to any one of claims 9, 11, 14 and 15.

**FIGURE 1**

EP 0 892 068 A1

~ 550 kb.

| PUBLIC MARKERS | WI13831 | D8S1413 | D8S277 | D8S561 |
|---|---|---|---|---|

BACs

H0287B09

B0228F07

B0189E08

B0463F01

| BAC INSERT SIZE (kb) | 150 | 230 | 200 | 130 |
|---|---|---|---|---|

| BIALLELIC MARKER | 8a-36 (262) | 99-123 (380) | 8a-56 (157) | 8a-26 (27) | 8a-14 (238) | 8a-67 (38) | 8a-77 (149) |
|---|---|---|---|---|---|---|---|
| ALLELIC FREQUENCY | 70/30 | 66/34 | 68/32 | 54/46 | 58/42 | 74/26 | 66/34 |

| MARKER/MARKER DISTANCE (kb) | 91 | 65 | 48 | 21 | 110 | 44 |
|---|---|---|---|---|---|---|

FIGURE 2

| BIALLELIC MARKER | | AMPLIFICATION PRIMERS 5'->3' § | POLYMORPHIC BASE * |
|---|---|---|---|
| 4-8a-36 (262) | PU | TGGGAGCTTAGAGAAGTG | *C/T Position 262* |
| | RP | CCATTCTTCCATTCCCTG | |
| 99-8a-123 (380) | PU | AAAGCCAGGACTAGAAGG | *C/T Position 380* |
| | RP | TATTCAGAAAGGAGTGGG | |
| 4-8a-56 (157) | PU | AAAGAGGAGTAAATGGGG | *C/T Position 157* |
| | RP | CTAAGGTGTTGTAGACAG | |
| 4-8a-26 (27) | PU | TACAGCCCTGTAAGACAC | *A/G Position 27* |
| | RP | TGAGGACTGCTAGGAAAG | |
| 4-8a-14 (238) | PU | TCTAACCTCTCATCCAAC | *C/T Position 238* |
| | RP | GACTGTATCCTTTGATGCAC | |
| 4-8a-67(38) | PU | AAGTTCACCTTCTCAAGC | *C/T Position 38* |
| | RP | TGAAAGAGTTTATTCTCTGG | |
| 4-8a-77 (149) | PU | TGTTGATTTACAGGCGGC | *C/G Position 149* |
| | RP | GGAAAGGTACTCATTCATAG | |

*§ All PU primers contain the following additional 5' sequence: TGTAAAACGACGGCCAGT*
*All RP primers contain the foolowing additional 5' sequence: CAGGAAACAGCTATGACC*
*\* Positions are based taking the 5' end of the specific sequence of the PU oligonucleotide as the first base of the amplicon.*

FIGURE 3

FIGURE 4

# FIGURE 5

| BIALLELIC MARKER | | AMPLIFICATION PRIMERS 5'->3' | POLYMORPHIC BASE * | MiS OLIGONUCLEOTIDE 5'->3' |
|---|---|---|---|---|
| 2-1-1 (396) | PU | GCCCAAAGAAGTGTGTTG | C/T Position 396 | GAAACATTAAACTCAAGGTTTCA |
| | RP | TTCCAAGCCATACGTGGTCTC | | |
| 2-1-14 (237) | PU | GTTCACTGAAGGGAACACAATTC | C/T Position 237 | AGAAGGATCTTTTAAGATACATA |
| | RP | TAGTAAGTCTAGGAGAGAAAAAATC | | |
| 2-1-48 (154) | PU | GTTTTATTTTATTGATGGGCTGTC | G/T Position 154 | TTTCAGTAAATATTTATTGAATAAGT |
| | RP | TTTTCTGCCTATGTGTATGGTGG | | |
| 99-1-24 (271) | PU | GCTGGCTAATAGAGTAATTG | C/G Position 271 | GTAATCTCCACATGTTGAGGGAG |
| | RP | TATAATCCATGCAGAGGGTGAAG | | |
| 99-1-7 (188) | PU | CAAGTCAGTGAGTAATCAG | A/G Position 188 | TTTAGGGAGCCTGTAAGTGGCTCGGA |
| | RP | AAGTAGTAACAAAGGAAGGCAG | | |

§ All PU primers contain the following additional 5' sequence: TGTAAAACGACGGCCAGT

All RP primers contain the foolowing additional 5' sequence: CAGGAAACAGCTATGACC

* Positions are based taking the 5' end of the specific sequence of the PU oligonucleotide as the first base of the amplicon.

MIS= Microsequencing

# FIGURE 6

| MARKER/MARKER | 4-8a-36(262) | 99-8a-123(380) | 4-8a-56(157) | 4-8a-26(27) | 4-8a-14(238) | 4-8a-67(38) | 4-8a-77(149) |
|---|---|---|---|---|---|---|---|
| DISTANCE (kb) | 91 | 65 | 48 | 21 | 110 | 44 | |
| 4-8a-36 (262) | | 1 | 8 | 7 | 8 | 8 | 7 |
| 99-8a-123 (380) | | | 22 | 14 | 13 | 4 | 7 |
| 4-8a-56 (157) | | | | 12 | 13 | 4 | 5 |
| 4-8a-26 (27) | | | | | 23 | 7 | 2 |
| 4-8a-14 (238) | | | | | | 6 | 2 |
| 4-8a-67 (38) | | | | | | | 21 |

EP 0 892 068 A1

# FIGURE 7

| BIALLELIC MARKER | POLYMORPHIC BASE * | MiS OLIGONUCLEOTIDE 5'->3' |
|---|---|---|
| 4-8a-36 (262) | C/T Position 262 | GATGACTGACTCCACGAATGGTA |
| 99-8a-123 (380) | C/T Position 380 | TTTCTCATCCTCACACCTCACTG |
| 4-8a-56 (157) | C/T Position 157 | AAGTTTTCCTTCTCTTCTGTAGA |
| 4-8a-26 (27) | A/G Position 27 | GATGCACTTTCCCATCTCAACAA |
| 4-8a-14 (238) | C/T Position 238 | GCAGGGAGCAGACCAGACATGAT |
| 4-8a-67(38) | C/T Position 38 | GCCAGTGAAATACAGACTTAATT |
| 4-8a-77 (149) | C/G Position 149 | GCTGTTCAGACTAAACTTGGAGA |

* Positions are based taking the 5' end of the specific sequence of the PU oligonucleotide as the first base of the amplicon.

MiS= Microsequencing

FIGURE 8

Two point lod (parametric analysis)

| MARKER | Distance (cM) | Z(lod)scores |
|---|---|---|
| D8S1742 | | -0.13 |
| D8S561 | 0.8 | -0.07 |

| | |
|---|---|
| *# of families analyzed* | *47* |
| *Total # of individuals genotyped* | *194* |
| *Total # of affected individuals genotyped* | *122* |

## FIGURE 9

| MARKER | Number | Distance in kb | Δ AF* (%) | chi2¤ | pvalue |
|---|---|---|---|---|---|
| 4-8a-36(262) | 1 | | 1,1 | 0,01 | 9,20E-01 |
| 99-8a-123(380) | 2 | 91 | -3,7 | 0,45 | 5,04E-01 |
| 4-8a-56(157) | 3 | 65 | 3,3 | 0,34 | 5,62E-01 |
| 4-8a-26(27) | 4 | 48 | 9,6 | 4,03 | 4,47E-02 |
| 4-8a-14(238) | 5 | 21 | 9,9 | 4,58 | 3,23E-02 |
| 4-8a-67(38) | 6 | 110 | -13 | 10,37 | 1,28E-03 |
| 4-8a-77(149) | 11 | 44 | -15,1 | 11,66 | 6,39E-04 |

*# alleles affected*      *360*
*# alleles non-affected*      *152*

*\* Δ AF= Difference in allele frequency between affected (prostate cancer) and non-affected individuals*

*¤ one freedom degree*

*The arrow indicates the region presented in Figure 10*

## FIGURE 10

| MARKER | Number | Distance in kb | Δ AF* (%) | chi2¤ | pvalue |
|---|---|---|---|---|---|
| 4-8a-67(38) | 6 | | -13 | 10,37 | 1,28E-03 |
| 4-8a-65(322) | 7 | 0,5 | 10,8 | 7,05 | 7,91E-03 |
| 4-8a-73(132) | 8 | 42,3 | -12,2 | 6,33 | 1,19E-02 |
| 4-8a-72(125) | 9 | 0,3 | 12 | 6,80 | 9,10E-03 |
| 4-8a-71(231) | 10 | 0,4 | 13,6 | 9,39 | 2,18E-03 |
| 4-8a-77(149) | 11 | 0,5 | -15,1 | 11,66 | 6,39E-04 |
| 4-8a-76(210) | 12 | 0,5 | -7,5 | 2,45 | 1,18E-01 |

*# alleles affected*      *360*
*# alleles non-affected*      *152*

*\* Δ AF= Difference in allele frequency between affected (prostate cancer) and non-affected individuals*
*¤ one freedom degree*

## FIGURE 11

| BIALLELIC MARKER | | AMPLIFICATION PRIMERS 5'->3' § | POLYMORPHIC BASE * | ALLELIC FREQUENCY | MiS OLIGONUCLEOTIDE 5'->3' |
|---|---|---|---|---|---|
| 4-8a-65 (322) | PU | GATTTAAGCTACGCTATTAG | *C/T Position 322* | 72/28 | GGTGCTGCTCAGCGGCTTGCACG |
| | RP | TGGCTCTGCATTTCTTCC | | | |
| 4-8a-73 (132) | PU | ATCGCTGGAACATTCTGG | *C/G Position 132* | 60/40 | GTTTTCCTTAAGATGTTACATGG |
| | RP | CTCTTGGTTAAACAGCAGTG | | | |
| 4-8a-72 (125) | PU | AAGATGTCTGTGATCGTG | *A/G Position 125* | 61/39 | GTTGGCTTTAAAAAGCAGGACAA |
| | RP | AAGGTTCTAAGGTAATCTGG | | | |
| 4-8a-71 (231) | PU | ACCATTATCAGCACAAGC | *A/G Position 231* | 65/35 | TCAGGTTCACTCGGATTAGAACA |
| | RP | TGGGTGTGTTCTGTAAAG | | | |
| 4-8a-76 (Del 210) | PU | GTAGAGCAGACATGCCGC | *Deletion Position 210* | 64/36 | TTGTAACATCCTTCTGGAAAACACTGT |
| | RP | GCAGTATCATAAGAACAGGG | | | |

*§ All PU primers contain the following additional 5' sequence: TGTAAAACGACGGCCAGT*

*All RP primers contain the foolowing additional 5' sequence: CAGGAAACAGCTATGACC*

*\* Positions are based taking the 5' end of the specific sequence of the PU oligonucleotide as the first base of the amplicon.*

*MiS= Microsequencing*

EP 0 892 068 A1

FIGURE 12

ALLELIC ASSOCIATION
3,000 MARKERS MAP

FIGURE 13

ALLELIC ASSOCIATION
20,000 MARKERS MAP

**FIGURE 14**

EP 0 892 068 A1

# FIGURE 15

## p   VALUE   DISTRIBUTION

| # aff | 150 | | | | | |
|---|---|---|---|---|---|---|
| # non aff | 150 | | | | | |
| pAi non aff | | 0 | 0,1 | 0,2 | 0,3 | 0,4 | 0,5 |
| Δ pAi | 0,05 | 8,7699E-05 | 0,06407752 | 0,14252002 | 0,19106311 | 0,21543442 | 0,22009395 |
| Δ pAi | 0,1 | 1,9149E-08 | 0,00060364 | 0,00467774 | 0,01023571 | 0,01382303 | 0,01382303 |
| Δ pAi | 0,15 | 3,0618E-12 | 1,3319E-06 | 3,8827E-05 | 0,0001478 | 0,0002343 | 0,00020218 |
| Δ pAi | 0,2 | 3,2153E-16 | 9,1413E-10 | 9,0305E-08 | 5,733E-07 | 9,6336E-07 | 5,733E-07 |
| Δ pAi | 0,25 | 2,0791E-20 | 2,2614E-13 | 6,2679E-11 | 5,873E-10 | 8,7113E-10 | 2,5396E-10 |
| Δ pAi | 0,3 | 7,8245E-25 | 2,152E-17 | 1,3261E-14 | 1,5189E-13 | 1,5189E-13 | 1,3261E-14 |
| Δ pAi | 0,35 | 1,6192E-29 | 7,9823E-22 | 8,4152E-19 | 9,1669E-18 | 4,2713E-18 | 5,5844E-20 |
| Δ pAi | 0,4 | 1,7336E-34 | 1,1282E-26 | 1,524E-23 | 1,1488E-22 | 1,524E-23 | 1,1282E-26 |

| # aff | 150 | | | | | |
|---|---|---|---|---|---|---|
| # non aff | 850 | | | | | |
| pAi non aff | | 0 | 0,1 | 0,2 | 0,3 | 0,4 | 0,5 |
| Δ pAi | 0,05 | 2,1561E-20 | 0,00994614 | 0,04896055 | 0,08358651 | 0,10417953 | 0,11025423 |
| Δ pAi | 0,1 | 2,0126E-39 | 5,571E-07 | 0,00010149 | 0,00058665 | 0,00119145 | 0,00139743 |
| Δ pAi | 0,15 | 1,1091E-58 | 2,7555E-13 | 8,462E-09 | 2,9851E-07 | 1,2395E-06 | 1,6229E-06 |
| Δ pAi | 0,2 | 3,2726E-78 | 2,1683E-21 | 3,2211E-14 | 1,1049E-11 | 1,111E-10 | 1,5638E-10 |
| Δ pAi | 0,25 | 4,9576E-98 | 4,4952E-31 | 6,5226E-21 | 3,1015E-17 | 2,5169E-16 | 1,1763E-15 |
| Δ pAi | 0,3 | 3,749E-118 | 3,6987E-42 | 8,129E-29 | 6,9335E-24 | 5,4331E-22 | 6,5657E-22 |
| Δ pAi | 0,35 | 1,383E-138 | 1,6797E-54 | 7,1058E-38 | 1,2938E-31 | 2,9415E-29 | 2,5869E-29 |
| Δ pAi | 0,4 | 2,435E-159 | 5,4915E-68 | 4,8846E-48 | 2,1003E-40 | 1,3332E-37 | 6,8178E-38 |

| # aff | 200 | | | | | |
|---|---|---|---|---|---|---|
| # non aff | 200 | | | | | |
| pAi non aff | | 0 | 0,1 | 0,2 | 0,3 | 0,4 | 0,5 |
| Δ pAi | 0,05 | 5,9233E-06 | 0,03250945 | 0,09039173 | 0,13111935 | 0,15260313 | 0,15678006 |
| Δ pAi | 0,1 | 8,649E-11 | 7,4765E-05 | 0,00109084 | 0,00302686 | 0,00447365 | 0,00447365 |
| Δ pAi | 0,15 | 8,0215E-16 | 2,3653E-08 | 2,0257E-06 | 1,1771E-05 | 2,1573E-05 | 1,7772E-05 |
| Δ pAi | 0,2 | 4,1762E-21 | 1,5375E-12 | 6,7374E-10 | 7,764E-09 | 1,5417E-08 | 7,764E-09 |
| Δ pAi | 0,25 | 1,1282E-26 | 2,525E-17 | 4,4025E-14 | 8,5532E-13 | 1,4423E-12 | 2,8149E-13 |
| Δ pAi | 0,3 | 1,4722E-32 | 1,1488E-22 | 5,8424E-19 | 1,4886E-17 | 1,4886E-17 | 5,8424E-19 |
| Δ pAi | 0,35 | 8,6169E-39 | 1,4784E-28 | 1,5457E-24 | 3,6958E-23 | 1,3394E-23 | 4,197E-26 |
| Δ pAi | 0,4 | 2,0885E-45 | 5,2308E-35 | 7,6438E-31 | 1,1224E-29 | 7,6438E-31 | 5,2308E-35 |

| # aff | 200 | | | | | |
|---|---|---|---|---|---|---|
| # non aff | 500 | | | | | |
| pAi non aff | | 0 | 0,1 | 0,2 | 0,3 | 0,4 | 0,5 |
| Δ pAi | 0,05 | 1,0628E-12 | 0,00789803 | 0,03942584 | 0,06867566 | 0,08621572 | 0,09083704 |
| Δ pAi | 0,1 | 3,4525E-24 | 4,4217E-07 | 5,6883E-05 | 0,00031976 | 0,0006363 | 0,00070881 |
| Δ pAi | 0,15 | 5,9036E-36 | 4,3025E-13 | 3,3635E-09 | 9,2134E-08 | 3,319E-07 | 3,5871E-07 |
| Δ pAi | 0,2 | 4,7325E-48 | 1,5566E-20 | 1,0346E-14 | 1,7218E-12 | 1,1512E-11 | 1,0047E-11 |
| Δ pAi | 0,25 | 1,6694E-60 | 3,5436E-29 | 2,0473E-21 | 2,2178E-18 | 1,1498E-17 | 1,3524E-17 |
| Δ pAi | 0,3 | 2,4613E-73 | 7,2498E-39 | 3,0748E-29 | 2,0601E-25 | 3,4525E-24 | 7,4807E-25 |
| Δ pAi | 0,35 | 1,4447E-86 | 1,6945E-49 | 3,9559E-38 | 1,4118E-33 | 2,662E-32 | 1,4118E-33 |
| Δ pAi | 0,4 | 3,214E-100 | 5,3051E-61 | 4,7325E-48 | 7,1282E-43 | 1,0691E-41 | 7,2652E-44 |

| # aff | 500 | | | | | |
|---|---|---|---|---|---|---|
| # non aff | 500 | | | | | |
| pAi non aff | | 0 | 0,1 | 0,2 | 0,3 | 0,4 | 0,5 |
| Δ pAi | 0,05 | 8,0004E-13 | 0,00072323 | 0,00741965 | 0,0169842 | 0,02371865 | 0,02516449 |
| Δ pAi | 0,1 | 1,0695E-24 | 3,7948E-10 | 2,4176E-07 | 2,7579E-06 | 6,9679E-06 | 6,9679E-06 |
| Δ pAi | 0,15 | 3,813E-37 | 1,0719E-18 | 5,8344E-14 | 4,2622E-12 | 1,8601E-11 | 1,1611E-11 |
| Δ pAi | 0,2 | 2,9626E-50 | 5,0895E-29 | 1,6881E-22 | 6,9321E-20 | 3,7441E-19 | 6,9321E-20 |
| Δ pAi | 0,25 | 4,2697E-64 | 7,2043E-41 | 7,7528E-33 | 1,194E-29 | 4,3462E-29 | 7,6438E-31 |
| Δ pAi | 0,3 | 9,6976E-79 | 3,9328E-54 | 6,3017E-45 | 1,9429E-41 | 1,9429E-41 | 6,3017E-45 |
| Δ pAi | 0,35 | 2,911E-94 | 8,8513E-69 | 8,7879E-59 | 2,3478E-55 | 1,8839E-56 | 1,1206E-62 |
| Δ pAi | 0,4 | 9,505E-111 | 7,7199E-85 | 1,8063E-74 | 1,4484E-71 | 1,8063E-74 | 7,7199E-85 |

| # aff | 500 | | | | | |
|---|---|---|---|---|---|---|
| # non aff | 1000 | | | | | |
| pAi non aff | | 0 | 0,1 | 0,2 | 0,3 | 0,4 | 0,5 |
| Δ pAi | 0,05 | 6,4766E-24 | 5,7827E-05 | 0,00172627 | 0,00551541 | 0,00882876 | 0,00978249 |
| Δ pAi | 0,1 | 6,5309E-47 | 3,065E-14 | 1,0301E-09 | 4,3205E-08 | 1,8833E-07 | 2,2731E-07 |
| Δ pAi | 0,15 | 1,1969E-70 | 2,0716E-27 | 3,7441E-19 | 4,6626E-16 | 6,9719E-15 | 6,9719E-15 |
| Δ pAi | 0,2 | 3,3252E-95 | 1,1636E-43 | 1,6614E-31 | 8,5632E-27 | 4,1421E-25 | 1,9885E-25 |
| Δ pAi | 0,25 | 1,227E-120 | 1,7683E-62 | 1,5329E-46 | 3,1722E-40 | 8,6765E-39 | 3,6071E-39 |
| Δ pAi | 0,3 | 5,303E-147 | 1,526E-83 | 4,2697E-64 | 2,5968E-56 | 3,9328E-54 | 2,5968E-56 |
| Δ pAi | 0,35 | 2,36E-174 | 1,184E-106 | 4,5658E-84 | 4,7426E-75 | 4,2624E-73 | 4,0958E-77 |
| Δ pAi | 0,4 | 9,446E-203 | 1,082E-131 | 2,137E-106 | 1,8014E-96 | 3,3252E-95 | 6,725E-102 |

# aff                     affected individuals

# non aff             non affected individuals

pAi  non aff      allele frequency in non affected individuals

Δ pAi                 % Difference in allele frequency between affected and non-affected individuals

EP 0 892 068 A1

European Patent
Office

# EUROPEAN SEARCH REPORT

Application Number

EP 97 40 1740

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | FINCHAM J R S: "Chapter 5.1: Mutation, the nature of alleles and the definition of the gene. In: GENETICS" 1983 , JOHN WRIGHT & SONS LTD , BRISTOL, ENGLAND XP002050817 * page 124 – page 126 * | 24 | C12Q1/68 |
| Y | SYVANEN AC ET AL: "Identification of individuals by analysis of biallelic DNA markers, using PCR and solid-phase minisequencing. " AMERICAN JOURNAL OF HUMAN GENETICS, vol. 52, no. 1, 1993, pages 46-59, XP002050638 * the whole document * | 1-11, 16-18, 21-24 | |
| D,Y | KIM UJ ET AL: "Construction and characterization of a human bacterial artificial chromosome library" GENOMICS, vol. 34, 1996, pages 213-218, XP002050639 * the whole document * | 1-11, 16-18, 21-24 | |
| Y | CHEE M ET AL: "Assessing genetic information with high-density DNA arrays" SCIENCE, vol. 274, 1996, pages 610-614, XP002050640 * the whole document * | 1,3,5-9, 16-22 | TECHNICAL FIELDS SEARCHED (Int.Cl.6) C12Q |
| Y | WANG D ET AL: "Towards a third generation genetic map of the human genome based on biallelic polymorphisms." AMERICAN JOURNAL OF HUMAN GENETICS, vol. 59, 1996, page A3 XP002050641 * the whole document * | 1,3,5-9, 16-22 | |

-/--

The present search report has been drawn up for all claims

| Place of search | Date of completion of the search | Examiner |
|---|---|---|
| THE HAGUE | 19 December 1997 | Knehr, M |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

                                             
& : member of the same patent family, corresponding
  document

EPO FORM 1503 03.82 (P04C01)

| European Patent Office | EUROPEAN SEARCH REPORT | Application Number<br>EP 97 40 1740 |
|---|---|---|

## DOCUMENTS CONSIDERED TO BE RELEVANT

| Category | Citation of document with indication. where appropriate, of relevant passages | Relevant to claim | CLASSIFICATION OF THE APPLICATION (Int.Cl.6) |
|---|---|---|---|
| Y | COX DR ET AL: "Assessing mapping progress in the human genome project" SCIENCE, vol. 265, 1994, pages 2031-2032, XP002050642 * the whole document * | 1,3-9, 16,24 | |
| Y | LANGE K AND SOBEL E: "A random walk method for computing genetic location scores." AMERICAN JOURNAL OF HUMAN GENETICS, vol. 49, no. 6, 1991, pages 1320-1334, XP002050643 * the whole document * | 1,3,4, 7-11,24 | |
| Y | RISCH N ET AL: "The future of genetic studies of complex human diseases" SCIENCE, vol. 273, 1996, pages 1516-1517, XP002050644 * the whole document * | 8-11,24 | |
| D,A | HUDSON TJ ET AL: "An STS-based map of the human genome" SCIENCE, vol. 270, 1995, pages 1945-1954, XP002050645 * the whole document * | 1,3-7 | TECHNICAL FIELDS SEARCHED (Int.Cl.6) |
| D,A | SCHULER GD ET AL: "A gene map of the human genome" SCIENCE, vol. 274, 1996, pages 540-546, XP002050646 * the whole document * | 1,5,6 | |

-/--

The present search report has been drawn up for all claims

| Place of search | Date of completion of the search | Examiner |
|---|---|---|
| THE HAGUE | 19 December 1997 | Knehr, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

# EUROPEAN SEARCH REPORT

Application Number

EP 97 40 1740

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,X | KRUGLYAK L: "The use of a genetic map of biallelic markers in linkage studies" NATURE GENETICS, vol. 17, no. 1, 1997, pages 21-24, XP002050647 * the whole document * | 1,3-9, 16,24 | |
| | | | TECHNICAL FIELDS SEARCHED (Int.Cl.6) |

The present search report has been drawn up for all claims

| Place of search | Date of completion of the search | Examiner |
|---|---|---|
| THE HAGUE | 19 December 1997 | Knehr, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)